# EUROPÄISCHE PATENTANMELDUNG

(11) **EP 1 975 165 A1**
(43) Veröffentlichungstag der Anmeldung: **01.10.2008**
(21) Anmeldenummer: 07105053.8
(22) Anmeldetag: 27.03.2007
(51) Int. Cl.: C07D 401/06, C07D 401/14, C07D 417/14, C07D 419/14, C07D 495/04, A61K 31/42, A61P 7/02

(54) **Substituierte Pyrrolidinamide, deren Herstellung und deren Verwendung als Arzneimittel**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Gegenstand der vorliegenden Erfindung sind neue substituierte Pyrrolidinamide der allgemeinen Formel (I)

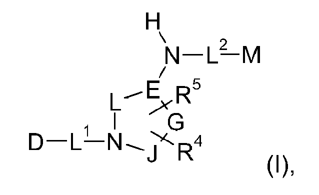

in denen D, L, E, G, J, M, L¹, L², R⁴ und R⁵ wie in der Beschreibung definiert sind, deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

## Beschreibung

Gegenstand der vorliegenden Erfindung sind neue substituierte Pyrrolidinamide der allgemeinen Formel (I) 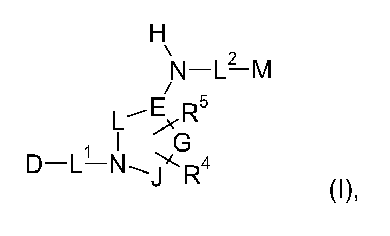 deren Tautomere, deren Enantiomere, deren Diastereomere, deren Gemische und deren Salze, insbesondere deren physiologisch verträgliche Salze mit anorganischen oder organischen Säuren oder Basen, welche wertvolle Eigenschaften aufweisen.

Die Verbindungen der obigen allgemeinen Formel (I) sowie deren Tautomere, deren Enantiomere, deren Diastereomere, deren Gemische und deren Salze, insbesondere deren physiologisch verträgliche Salze mit anorganischen oder organischen Säuren oder Basen, und deren Stereoisomere weisen wertvolle pharmakologische Eigenschaften auf, insbesondere eine antithrombotische Wirkung und eine Faktor Xa-inhibierende Wirkung.

Gegenstand der vorliegenden Anmeldung sind neue Verbindungen der obigen allgemeinen Formel (I), deren Herstellung, die die pharmakologisch wirksamen Verbindungen enthaltenden Arzneimittel, deren Herstellung und Verwendung.

Eine 1. Ausführungsform der vorliegenden Erfindung umfasst diejenigen Verbindungen der allgemeinen Formel (I), in denen
- D: ein substituiertes bicyclisches Ringsystem der Formel (IIa), (IIb) oder (IIc) 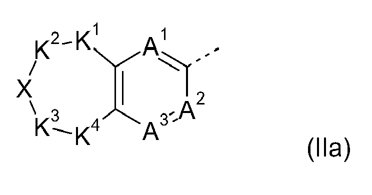 oder 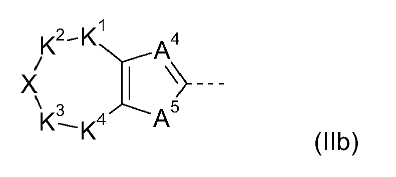 oder 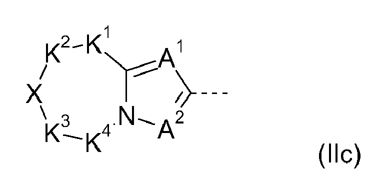 darstellt, in dem
K¹ und K⁴ jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c} jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe, eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁-₅-alkyl-, C₁₋₅-Alkylamino-C₁₋₅-alkyl-, Di-(C₁₋₅-alkyl)-amino-C₁₋₅-alkyl-, C₄₋₇Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇Cycloalkyleniminocarbonyl-C₀₋₅-alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon- oder [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem N-Atom durch eine -CO-Gruppe ersetzt sein kann, und/oder dessen Iminogruppen jeweils durch eine C₁₋₃-Alkyl- oder C₁₋₃-Alkylcarbonyl-gruppe substituiert sein können, und/oder
bei dem das Schwefelatom zu einer Sulfoxid oder Sulfongruppe oxidiert sein kann,
- K² und K³: jeweils unabhängig voneinander eine -CH₂-, -CHR^{8a}-, -CR^{8b}R^{8c}- oder eine -C(O)-Gruppe bedeuten, wobei
R^{8a}/R^{8b}/R^{8c} jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem Stickstoffatom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder
bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (I) entfernt sein darf, und
insgesamt in Formel (IIa) oder (IIb) oder (IIc) maximal vier Reste ausgewählt aus R^{7a}, R^{7b}, R^{7c}, R^{8a}, R^{8b} und R^{8c} vorhanden sein dürfen, und
- X: ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkylsulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅-Alkoxycarbonylgruppe substituiert sein können, oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
A⁴ entweder N oder CR¹² bedeutet,
A⁵ NH, Schwefel oder Sauerstoff bedeutet, wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Phenyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
- -L-E-G-J-: eine -C-C-C-C-Gruppe bedeutet, die durch R⁴ und R⁵ substituiert sein kann, und
- L¹: eine -C(O)-Gruppe bedeutet, und
- R⁴: ein Wasserstoffatom oder
eine geradkettige oder verzweigte C₁₋₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N-(*C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet,
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyl-oxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₅-Alkyloxycarbonyl-, Carboxy-, Methyl-, Hydroxy-, Methoxy- oder Aminosubstituiert sein können, und
die vorgenannten Phenyl- oder Heteroarylreste gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O- Bindung bilden, ausgeschlossen ist, und
- R⁵: ein Wasserstoffatom, eine C₁₋₅ Alkyl-, C₂₋₅ Alkenyl- oder C₂₋₅ Alkinyl- oder eine Phenyl-C₀₋₅Alkylgruppe bedeutet, wobei die Alkylgruppe durch eine Hydroxy-, Methoxy-, Hydroxycarbonyl- oder C₁₋₅Alkoxycarbonyl-gruppe substituiert sein kann,
oder sofern R⁵ mit G verknüpft ist auch eine Hydroxy- oder Methoxy-gruppe darstellen kann, oder
- R⁴ und R⁵: sofern sie an das selbe Kohlenstoffatom gebunden sind, zusammen mit dem Kohlenstoffatom eine -C=O Gruppe, oder eine -CF₂ Gruppe bilden können, oder
- R⁴ und R⁵: sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus oder einen einfach ungesättigten 5-7 gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, -N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂-O-Gruppe ersetzt sein können, und/oder
wobei 1 bis 3 Kohlenstoffatome dieser 3-7-gliedrigen Cyclen gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O- Bindung bilden,
ausgeschlossen ist, und
- L²: eine -C(O)-Gruppe bedeutet, und
- M: einen gegebenenfalls durch R² und R³ substituierten Phenyl-, Pyridyl-, Thienyl- oder Furyl-Ring bedeutet, in dem
R² ein Fluor-, Chlor-, Brom- oder Jod-atom oder eine Methyl-, Ethyl-, Vinyl-, Methoxy-, Ethinyl-, Cyano- oder -C(O)NH₂-Gruppe darstellt, und
R³ ein Wasserstoff-, Fluor-, Chlor-, Brom- oder Jod-atom oder eine Hydroxy-, Methoxy-, Trifluormethoxy-gruppe, oder eine gegebenenfalls durch Fluoratome substituierte C₁₋₃-Alkyl-gruppe, oder eine Cyano-, Amino- oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

Beispiele für monocyclische Heteroarylgruppen sind die Pyridyl-, *N*-Oxy-pyridyl-, Pyrazolyl-, Pyridazinyl-, Pyrimidinyl-, Pyrazinyl-, [1,2,3]Triazinyl-, [1,3,5]Triazinyl-, [1,2,4]Triazinyl-, Pyrrolyl-, Imidazolyl-, [1,2,4]Triazolyl-, [1,2,3]Triazolyl-, Tetrazolyl-, Furanyl-, Isoxazolyl-, Oxazolyl-, [1,2,3]Oxadiazolyl-, [1,2,4]Oxadiazolyl-, Furazanyl-, Thienyl-, Thiazolyl-, Isothiazolyl-, [1,2,3]Thiadiazolyl-, [1,2,4]Thiadiazolyl- oder [1,2,5]Thiadiazolyl-Gruppe.

Beispiele für bicyclische Heteroarylgruppen sind die Benzimidazolyl, Benzofuranyl-, Benzo[*c*]furanyl-, Benzothiophenyl-, Benzo[*c*]thiophenyl-, Benzothiazolyl-, Benzo[*c*]isothiazolyl-, Benzo[*d*]isothiazolyl-, Benzooxazolyl-, Benzo[*c*]isoxazolyl-, Benzo[*d*]isoxazolyl-, Benzo[1,2,5]oxadiazolyl-, Benzo[1,2,5]thiadiazolyl-, Benzo[1,2,3]thiadiazolyl-, Benzo[*d*][1,2,3]triazinyl-, Benzo[1,2,4]triazinyl-, Benzotriazolyl-, Cinnolinyl-, Chinolinyl-, N-Oxy-chinolinyl-, Isochinolinyl-, Chinazolinyl-, N-Oxy-chinazolinyl-, Chinoxalinyl-, Phthalazinyl-, Indolyl-, Isoindolyl- oder 1-Oxa-2,3-diaza-indenyl-Gruppe.

Beispiele für die voranstehend in den Definitionen erwähnten C₁₋₆-Alkylgruppen sind die Methyl-, Ethyl-, 1-Propyl-, 2-Propyl-, n-Butyl-, *sec*-Butyl-, *tert*-Butyl-, 1-Pentyl-, 2-Pentyl-, 3-Pentyl-, *neo*-Pentyl-, 3-Methyl-2-butyl-, 1-Hexyl-, 2-Hexyl-, 3-Hexyl, 3-Methyl-2-pentyl-, 4-Methyl-2-pentyl-, 3-Methyl-3-pentyl-, 2-Methyl-3-pentyl-, 2,2-Dimethyl-3-butyl- oder 2,3-Dimethyl-2-butyl-Gruppe.

Beispiele für die voranstehend in den Definitionen erwähnten C₁₋₅-Alkyloxygruppen sind die Methyloxy-, Ethyloxy-, 1-Propyloxy-, 2-Propyloxyn-Butyloxy-, *sec*-Butyloxy-, *tert*-Butyloxy-, 1-Pentyloxy-, 2-Pentyloxy-, 3-Pentyloxy- oder *neo*-Pentyloxy-Gruppe.

Beispiele für die voranstehend in den Definitionen erwähnten C₂₋₅-Alkenylgruppen sind die Ethenyl-, 1-Propen-1-yl-, 2-Propen-1-yl-, 1-Buten-1-yl-, 2-Buten-1-yl-, 3-Buten-1-yl-, 1-Penten-1-yl-, 2-Penten-1-yl-, 3-Penten-1-yl-, 4-Penten-1-yl-, 1-Hexen-1-yl-, 2-Hexen-1-yl-, 3-Hexen-1-yl-, 4-Hexen-1-yl-, 5-Hexen-1-yl-, But-1-en-2-yl-, But-2-en-2-yl-, But-1-en-3-yl-, 2-Methyl-prop-2-en-1-yl-, Pent-1-en-2-yl-, Pent-2-en-2-yl-, Pent-3-en-2-yl-, Pent-4-en-2-yl-, Pent-1-en-3-yl-, Pent-2-en-3-yl-, 2-Methyl-but-1-en-1-yl-, 2-Methyl-but-2-en-1-yl-, 2-Methyl-but-3-en-1-yl- oder 2-Ethyl-prop-2-en-1-yl -Gruppe,

Beispiele für die voranstehend in den Definitionen erwähnten C₂₋₅-Alkinylgruppen sind die Ethinyl-, 1-Propinyl-, 2-Propinyl-, 1-Butin-1-yl-, 1-Butin-3-yl-, 2-Butin-1-yl-, 3-Butin-1-yl-, 1-Pentin-1-yl-, 1-Pentin-3-yl-, 1-Pentin-4-yl-, 2-Pentin-1-yl-, 2-Pentin-3-yl-, 3-Pentin-1-yl-, 4-Pentin-1-yl-, 2-Methyl-1-butin-4-yl-, 3-Methyl-1-butin-1-yl- oder 3-Methyl-1-butin-3-yl-Gruppe.

Eine 2. Ausführungsform der vorliegenden Erfindung umfasst diejenigen Verbindungen der allgemeinen Formel (I) in denen D, E, G, J, L, L¹, L² und M wie in Ausführungsform 1 beschrieben definiert sind, und in der
- R⁴: ein Wasserstoffatom oder
eine geradkettige oder verzweigte C₁₋₆-Alkylgruppe bedeutet,
wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₂-alkyloxygruppe, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₅-Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
- R⁵: ein Wasserstoffatom oder eine C₁₋₅ Alkyl-, Allyl-, Propargyl- oder Benzylgruppe bedeutet, oder sofern R⁵ mit G verknüpft ist, auch eine Hydroxy- oder Methoxy-gruppe darstellen kann, oder
- R⁴ und R⁵: sofern sie an das selbe Kohlenstoffatom gebunden sind, zusammen mit dem Kohlenstoffatom eine -C=O-Gruppe, oder eine -CF₂- Gruppe bilden können, oder
- R⁴ und R⁵: sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-,-N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyden zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂O-Gruppe ersetzt sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
deren Tautomere, deren Enantiomere, deren Diastereomere, deren Gemische und deren Salze.

Eine 3. Ausführungsform der vorliegenden Erfindung umfasst diejenigen Verbindungen der allgemeinen Formel (I), in denen E, G, J, L, L¹, L², M, R⁴ und R⁵ wie in den Ausführungsformen 1 oder 2 beschrieben definiert sind, und in der
- D: ein substituiertes bicyclisches Ringsystem der Formel (IIa) oder (IIb) 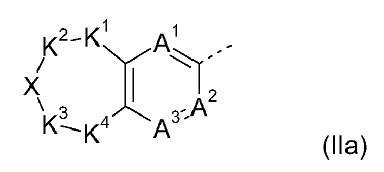 oder 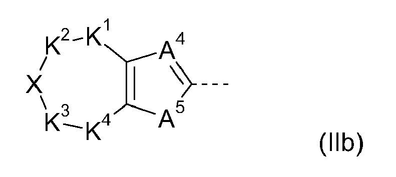 darstellt, in dem
K¹ und K⁴ jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c} jeweils unabhängig voneinander ein Fluoratom, eine Hydroxy-, C₁-5-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer -CF₂-Gruppe, oder
zwei Reste R^{7b}/R^{7c} zusammen mit dem Ringkohlenstoffatom einen 3-gliedrigen Carbocyclus bilden können,
mit der Maßgabe, dass K¹ und K⁴ gleichzeitig eine Bindung bedeuten, ausgeschlossen ist, und
K² und K³ jeweils unabhängig voneinander eine -CH₂-, -CHR^{8a}-, -CR^{8b}R^{8c}- oder eine -C(O)- Gruppe bedeuten, wobei
R^{8a}/R^{8b}/R^{8c} jeweils unabhängig voneinander eine C₁₋₅-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt in den Formeln (IIa) oder (IIb) maximal vier Reste ausgewählt aus R^{7a} R^{7b}, R^{7c}, R^{8a}, R^{8b} und R^{8c} vorhanden sein dürfen, und
- X: ein Sauerstoff- oder Schwefelatom, eine -CF₂- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
- A¹: entweder N oder CR¹⁰ bedeutet,
- A²: entweder N oder CR¹¹ bedeutet,
- A³: entweder N oder CR¹² bedeutet,
- A⁴: entweder N oder CR¹² bedeutet,
- A⁵: NH, Schwefel oder Sauerstoff bedeutet,
wobei R¹⁰, R¹¹ und R¹² jeweils unabhängig voneinander
ein Wasserstoff-, Fluor-, Chlor-, Brom- oder Iodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkyleniminogruppe bedeuten, deren Tautomere, deren Enantiomere, deren Diastereomere, deren Gemische und deren Salze.

Eine 4. Ausführungsform der vorliegenden Erfindung umfasst diejenigen Verbindungen der Ausführungsformen 1, 2 oder 3, in denen
- X: eine NR¹-Gruppe bedeutet, in der
R¹ ein Wasserstoffatom oder eine C₁₋₅-Alkyl-, Allyl- oder Cyclopropylgruppe bedeutet, und
- A¹: CR¹⁰ bedeutet,
- A²: CR¹¹ bedeutet,
- A³: CR¹² bedeutet,
- A⁴: entweder N oder CR¹² bedeutet,
- A⁵: Schwefel bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

Eine 5. Ausführungsform der vorliegenden Erfindung umfasst diejenigen Verbindungen der Ausführungsformen 1, 2, 3 oder 4, in denen
- D: ein substituiertes bicyclisches Ringsystem der Formel 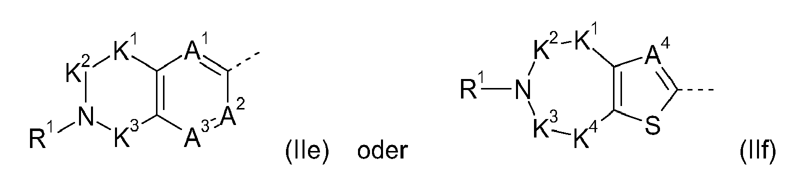 darstellt, in dem
K¹ eine -CH₂-, -CHR^{7a}-, oder eine -CR^{7b}R^{7c}- Gruppe bedeutet, und
K² und K³ jeweils unabhängig voneinander eine -CH₂-, -CHR^{8a}-, oder eine - CR^{8b}R^{8c}- Gruppe bedeuten, wobei
R^{8a}/R^{8b}/R^{8c} jeweils unabhängig voneinander eine C₁₋₅-Alkylgruppe bedeutet, und
K⁴ eine Bindung, eine -CH₂-, -CHR^{7a}-, -CR^{7b}R^{7c}- oder eine -C(O)-Gruppe bedeutet,
wobei
R^{7a} eine C₁₋₅-Alkylgruppe bedeutet und
R^{7b}/R^{7c} jeweils unabhängig voneinander eine Hydroxy-, C₁₋₅-Alkyloxy- oder eine C₁₋₅-Alkylgruppe bedeutet,
wobei nicht gleichzeitig beide Reste R^{7b}/R^{7c} über ein Sauerstoffatom an das Ringkohlenstoffatom gebunden sein können, und
insgesamt in den Formeln (IIe) oder (IIf) maximal vier Reste ausgewählt aus R^{7a} R^{7b}, R^{7c}, R^{8a}, R^{8b} und R^{8c} vorhanden sein dürfen, und
- R¹: ein Wasserstoffatom oder eine C₁₋₃-Alkyl-, Allyl- oder Cyclopropylgruppe bedeutet, und in denen
- A¹: CR¹⁰ bedeutet,
- A²: CR¹¹ bedeutet,
- A³: CR¹² bedeutet,
- A⁴: entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
- -L-E-G-J-: eine -C-C-C-C-Gruppe bedeutet, die durch R⁴ und R⁵ substituiert sein kann, und
- R⁴: ein Wasserstoffatom oder
eine geradkettige oder verzweigte C₁₋₃-Alkylgruppe bedeutet,
wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
sofern R⁴ mit G verknüpft ist, auch ein Fluoratom oder eine Hydroxy-, Methoxy-, C₃₋₅-Alkenyl-oxy-, C₂₋₅-Alkyl-oxy-, C₃₋₆-Cycloalkyl-oxy-, Benzyloxy-, C₁₋₅-Alkylaminocarbonyloxy-, Di(C₁₋₅-alkyl)aminocarbonyloxy- oder eine C₄₋₇-Cycloalkyleniminocarbonyloxygruppe darstellen kann,
mit der Maßgabe,
dass zwei Heteroatome aus der Gruppe Sauerstoff und Stickstoff durch genau eine gegebenenfalls substituierte -CH₂-Gruppe voneinander getrennt sind,
ausgeschlossen ist, und
- R⁵: ein Wasserstoffatom oder eine C₁₋₅ Alkyl-, Allyl-, Benzyl- oder Phenylgruppe bedeutet, oder sofern R⁵ mit G verknüpft ist, auch eine Hydroxy- oder Methoxy-gruppe darstellen kann, oder
- R⁴ und R⁵: sofern sie an das selbe Kohlenstoffatom gebunden sind, zusammen mit dem Kohlenstoffatom eine -C=O-Gruppe, oder eine -CF₂- Gruppe bilden können, oder
- R⁴ und R⁵: sofern sie an das selbe Kohlenstoffatom oder an zwei benachbarte Kohlenstoffatome gebunden sind, zusammen mit dem oder den Kohlenstoffatomen einen 3-6-gliedrigen Carbocyclus bilden können,
wobei vier direkt benachbarte Kohlenstoffkettenglieder dieser C₅₋₆-Carbocyclen zusammen durch eine -O-CH₂-CH₂O-Gruppe ersetzt sein können,
deren Tautomere, deren Enantiomere, deren Diastereomere, deren Gemische und deren Salze.

Eine 6. Ausführungsform der vorliegenden Erfindung umfasst diejenigen Verbindungen der Ausführungsformen 1, 2, 3, 4 oder 5, in denen
- D: ein substituiertes bicyclisches Ringsystem der Formel 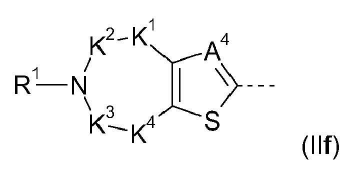 darstellt, in dem
K¹ eine -CH₂-, -CHR^{7a}-, oder eine -CR^{7b}R^{7c}- Gruppe bedeutet, und
K² und K³ jeweils unabhängig voneinander eine -CH₂-, -CHR^{8a}-, oder eine - CR^{8b}R^{8c}- Gruppe bedeuten, wobei
R^{8a}/R^{8b}/R^{8c} jeweils unabhängig voneinander eine C₁₋₅-Alkylgruppe bedeutet, und
K⁴ eine Bindung, eine -CH₂-, -CHR^{7a}-, oder eine -CR^{7b}R^{7c}- Gruppe
bedeutet,
wobei
R^{7a} eine C₁₋₅-Alkylgruppe bedeutet, und
R^{7b}/R^{7c} jeweils unabhängig voneinander eine Hydroxy-, C₁₋₅-Alkyloxy- oder eine C₁₋₅-Alkylgruppe bedeutet,
wobei nicht gleichzeitig beide Reste R^{7b}/R^{7c} über ein Sauerstoffatom an das Ringkohlenstoffatom gebunden sein können,
und
insgesamt in der Formel (IIf) maximal vier Reste ausgewählt aus R^{7a}, R^{7b}, R^{7c}, R^{8a}, R^{8b} und R^{8c} vorhanden sein dürfen, und
- R¹: ein Wasserstoffatom oder eine C₁₋₃-Alkyl- oder Cyclopropylgruppe bedeutet, und in denen
- A¹: CR¹⁰ bedeutet,
- A²: CR¹¹ bedeutet,
- A³: CR¹² bedeutet,
- A⁴: entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

Eine 7. Ausführungsform der vorliegenden Erfindung umfasst diejenigen Verbindungen der Ausführungsformen 1, 2, 3, 4, 5 oder 6, in denen
- M: einen Thiophen-2-yl-Ring der Formel 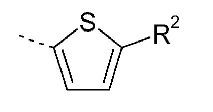 bedeutet, in dem
R² ein Chlor-, Bromatom oder eine Ethinyl-Gruppe darstellt,
deren Tautomere, deren Enantiomere, deren Diastereomere, deren Gemische und deren Salze.

Erfindungsgemäß erhält man die Verbindungen der allgemeinen Formel (1) nach an sich bekannten Verfahren, beispielsweise nach folgenden Verfahren:
(a) Die Herstellung einer Verbindung der allgemeinen Formel (111) 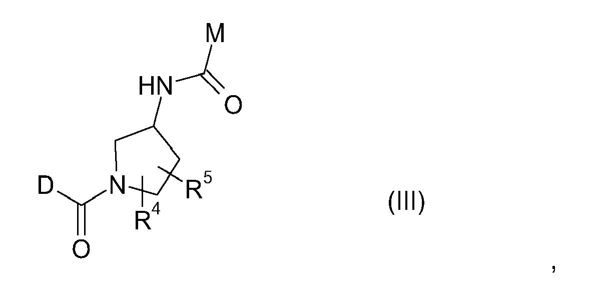 in der D, M und R¹ bis R⁵ wie in Ausführungsform 1 erwähnt definiert sind,
   und die gegebenenfalls an vorhandenen Amino-, Hydroxy-, Carboxy- oder Thiolgruppen durch gängige Schutzgruppen wie beispielsweise denen in T.W. Greene, P.G.M. Wuts in "Protective Groups in Organic Synthesis", Wiley, 1991 und 1999, beschriebenen geschützt sein kann, und deren Schutzgruppen in Literatur nach bekannter Methode abgespalten werden können,
   wird in den Ausführungsbeispielen beschrieben oder kann beispielsweise nach einem der folgenden Formelschemata 1 und 2 oder in Analogie zu den Syntheseverfahren, die in W02002/14308 oder W02006/114402 beschrieben sind, durchgeführt werden. 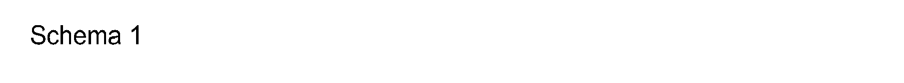 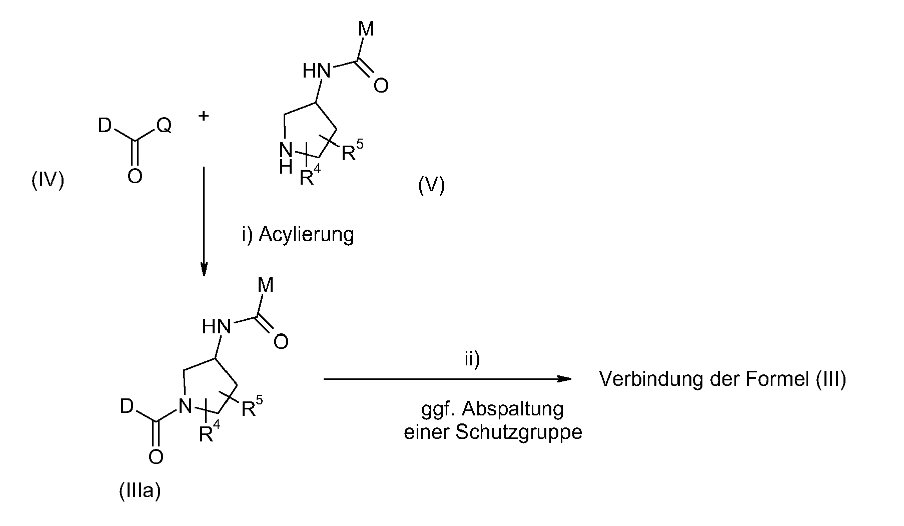 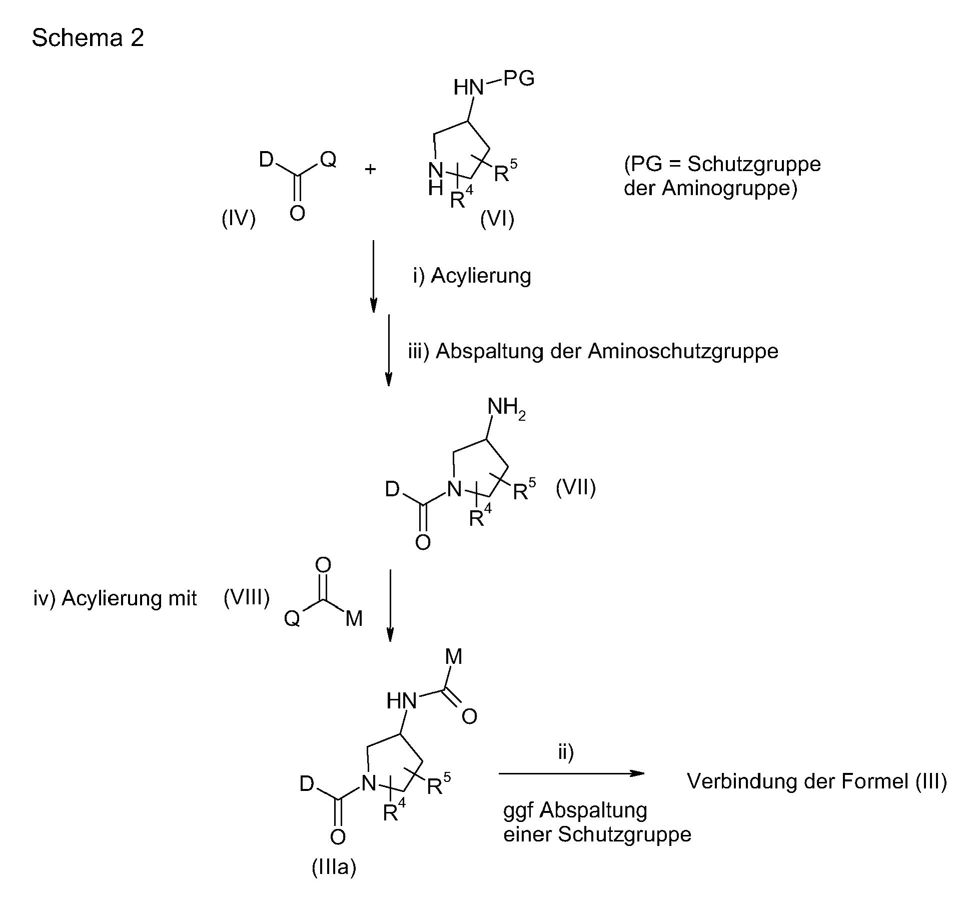 wobei
   Q eine Austrittsgruppe oder eine *in-situ* in eine Austrittsgruppe überführbare Gruppe wie beispielsweise ein Halogenatom, eine Hydroxy-, C₁₋₄-Alkyloxy-, Alkyloxycarbonyloxy-, Pentafluorphenyloxy-, 4-Nitrophenyloxy-, eine Trichlormethyl- oder Acyloxy-gruppe darstellt, und PG eine in Literatur bekannte Schutzgruppe der Aminofunktion wie beispielsweise eine tert.-Butoxycarbonyl-, Benzyloxycarbonyl- oder eine Trifluoracetyl-gruppe darstellt.
   Die in Schema 1 und 2 beschriebenen Reaktionsstufen i) -iv) können auf die in den Beispielen beschriebene Weise oder nach in Literatur bekannter Bedingungen beispielsweise wie folgt durchgeführt werden:
   i) Acylierung eines Amins (V) mit einer gegebenenfalls aktivierten Carbonsäure (IV) oder (VIII) :
      Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril, Dimethylformamid, Dimethylsulfoxid, Natronlauge oder Sulfolan gegebenenfalls in Gegenwart einer anorganischen oder organischen Base bei Temperaturen zwischen -20 und 200°C, vorzugsweise jedoch bei Temperaturen zwischen -10 und 100°C, durchgeführt.
      Die Acylierung kann jedoch auch mit der freien Säure gegebenenfalls in Gegenwart eines die Säure aktivierenden Mittels oder eines wasserentziehenden Mittels, beispielsweise in Gegenwart von Ethyl-1-ethoxy-1,2-dihydrochinolin-1-carboxylat, Chlorameisensäureisobutylester, Thionylchlorid, Trimethylchlorsilan, Chlorwasserstoff, Schwefelsäure, Methansulfonsäure, p-Toluolsulfonsäure, Phosphortrichlorid, Phosphorpentoxid, Propanphosphonsäurecycloanhydrid, *N*,*N*'-Dicyclohexylcarbodiimid, *N*,*N*'-Dicyclohexylcarbodiimid/Camphersulfonsäure, *N*,*N*'-Dicyclohexyl-carbodiimid/*N*-Hydroxysuccinimid oder 1-Hydroxy-benztriazol, *N*,*N*'-Carbonyldiimidazol, *O*-(Benzotriazol-1-yl*)-N,N,N',N'*-tetramethyl-uroniumtetrafluorborat/*N*-Methylmorpholin, *O*-(Benzotriazol-*1-yl)-N,N,N',N'-tetramethyl-uroniumtetrafluorborat*/*N-*Ethyldiisopropylamin, *O*-(7-Azabenzotriazol-1-yl)-*N*,*N*,*N*;*N*'-tetramethyluronium-hexafluorphosphat/N-Methylmorpholin, *O*-Pentafluorophenyl-*N*,*N*,*N'*,*N'*-tetramethyluroniumhexafluorophosphat/Triethylamin, *N*,*N'*-Thionyldiimidazol oder Triphenylphosphin/Tetrachlorkohlenstoff, gegebenenfalls unter Zusatz einer Hilfsbase wie Natronlauge, Cäsium-, Kalium- oder Natrium-carbonat oder -hydrogencarbonat oder einer Aminbase wie Pyridin, Triethylamin, N-Methylmorpholin oder Diisopropylethylamin bei Temperaturen zwischen -20 und 200°C, vorzugsweise jedoch bei Temperaturen zwischen -10 und 160°C, durchgeführt werden.
      Weitere Verfahren zur Amidkupplung sind beispielsweise in P.D. Bailey, I.D. Collier, K.M. Morgan in "Comprehensive Functional Group Interconversions", Vol. 5, Seite 257ff., Pergamon 1995, oder auch im Houben-Weyl Ergänzungsband 22, Thieme Verlag, 2003 und der dort zitierten Literatur beschrieben.
   ii) bzw. iii) Abspaltung einer Schutzgruppe
      Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.
      Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Tetrahydrofuran, Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.
      Die Abspaltung einer Schutzgruppe kann aber auch nach den in T.W. Greene, P.G.M. Wuts in "Protective Groups in Organic Synthesis", Wiley, 1991 und 1999, beschriebenen Verfahren durchgeführt werden.
(b) Die Bausteine der allgemeinen Formel 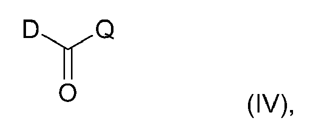 wobei Q eine Hydroxy- oder Alkyloxy-Gruppe darstellt, und
   in denen D wie in Ausführungsform 1 erwähnt definiert sind, und
   die gegebenenfalls an vorhandenen Amino-, Hydroxy-, Carboxy- oder Thiol-gruppen durch gängige Schutzgruppen, wie beispielsweise denen in T.W. Greene, P.G.M. Wuts in "Protective Groups in Organic Synthesis", Wiley, 1991 und 1999, beschriebenen geschützt sein können, und deren Schutzgruppen in Literatur nach bekannter Methode im Verlauf der Synthesesequenz zu Verbindungen der Formel (I) abgespalten werden können,
   sind aus der Literatur bekannt, oder deren Synthese wird in den Ausführungsbeispielen beschrieben, oder sie können beispielsweise nach in Literatur bekannter Syntheseverfahren oder in Analogie zu in Literatur bekannten Syntheseverfahren wie beispielsweise in DE3105858, JP04046139 bzw. in N. Haginoya et al. J. Med. Chem. 2004, 47(21), 5167, S. Komoriya et al. Bioorg. Med. Chem. 2006, 14, 1309, Ortar et al. Tetrahedron Lett. 1986, 3931 oder in J.M. Herbert et al., Tetrahedron. Lett 1998, 2421, beschrieben, hergestellt werden.
   Beispielsweise kann eine Verbindung der allgemeinen Formel (IV), in der D wie in Ausführungsform 1 erwähnt definiert sind, durch palladiumvermittelte Carboxylierung in Alkoholen oder Wasser aus Verbindungen der allgemeinen Formeln (X) oder (Xa) 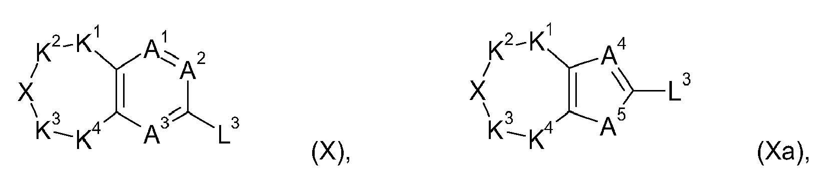 wobei
   L³ eine Austrittsgruppe oder eine *in-situ* in eine Austrittsgruppe überführbare Gruppe wie beispielsweise ein Halogenatom oder ein Trifluormethansulfonat darstellt, und
   in der A¹, A², A³, A⁴, A⁵, K¹, K², K³, K⁴ und X wie in Ausführungsform 1 erwähnt definiert sind, wie folgt hergestellt werden.
   Die Einführung einer Estergruppe aus Verbindungen der allgemeinen Formeln (X) oder (Xa) wird beispielsweise zweckmäßigerweise mit einem Alkohol durch katalytische Carbonylierung mit Kohlenmonoxid, beispielsweise unter einem Druck zwischen 0.5 und 100 bar, vorzugsweise jedoch zwischen 1 und 50 bar, zweckmäßigerweise in Gegenwart eines Katalysators wie beispielsweise Palladium(II)acetat, Tetrakis(triphenylphosphin)palladium(0) oder Dichlorbis(triphenylphosphin)palladium(II), zweckmäßigerweise in einem Lösungsmittel oder Lösungsmittelgemisch wie Wasser, Methanol, Ethanol, Isopropanol, Butanol, Pentan, Hexan, Cyclohexan, Heptan, Benzol, Toluol, Xylol, Ethylacetat, Methylpropionat, Glykol, Glykoldimethylether, Diethylenglykoldimethylether, Dioxan, Tetrahydrofuran, beispielsweise bei Temperaturen zwischen -30 und 250°C, vorzugsweise jedoch zwischen 0 und 150°C, durchgeführt.
(c) Die Bausteine der allgemeinen Formel 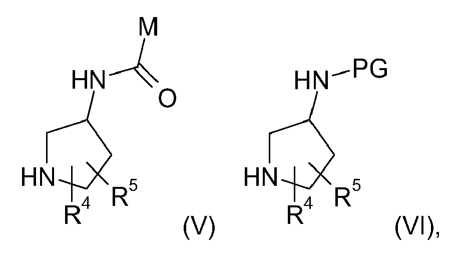 in denen M, R⁴ und R⁵ wie in Ausführungsform 1 erwähnt definiert sind, sind in der Literatur bekannt, oder deren Synthese wird in den Ausführungsbeispielen beschrieben, oder sie können beispielsweise nach in der Literatur bekannten Syntheseverfahren oder in Analogie zu in der Literatur bekannten Syntheseverfahren wie beispielsweise in W02006/114402 beschrieben, hergestellt werden.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, *tert.*-Butyl-, Trityl-, Benzyl- oder Tetrahydropyranyl-gruppe in Betracht.

Beispielsweise kommt als Schutzrest für eine Carboxylgruppe die Trimethylsilyl-, Methyl-, Ethyl-, *tert*.-Butyl-, Benzyl- oder Tetrahydropyranyl-gruppe in Betracht.

Beispielsweise kommt als Schutzrest für eine Amino-, Alkylamino- oder Iminogruppe die Acetyl-, Trifluoracetyl-, Benzoyl-, Ethoxycarbonyl-, *tert*.-Butoxycarbonyl-, Benzyloxycarbonyl-, Benzyl-, Methoxybenzyl- oder 2,4-Dimethoxybenzyl-gruppe, und für die Aminogruppe zusätzlich die Phthalylgruppe in Betracht.

Beispielsweise kommt als Schutzrest für eine Ethinylgruppe die Trimethylsilyl-, Diphenylmethylsilyl-, tert.Butyldimethylsilyl- oder eine 1-Hydroxy-1-methyl-ethylgruppe in Betracht.

Weitere Schutzgruppen die eingesetzt werden können und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxygruppe erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Ferner können die erhaltenen Verbindungen der allgemeinen Formel (I) in ihre Enantiomeren und/oder Diastereomeren aufgetrennt werden.

So lassen sich beispielsweise die erhaltenen Verbindungen der allgemeinen Formel (I), welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel (I) mit mindestes zwei asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch chromatographische Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel (I) in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel (I), falls diese eine Carboxygruppe enthalten, gegebenenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die Verbindungen der allgemeinen Formel (I) sowie deren Tautomere, deren Enantiomere, deren Diastereomere und deren physiologisch verträglichen Salze wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xahemmenden Wirkung, auf einer die aPTT-Zeit verlängernden Wirkung und auf einer Hemmwirkung auf verwandte Serinproteasen wie z. B. Urokinase, Faktor VIIa, Faktor IX, Faktor XI und Faktor XI I.

Die im Experimentellen Teil angeführten Verbindungen können auf ihre Wirkung auf die Hemmung des Faktors Xa wie folgt untersucht werden.

### Methodik

Enzymkinetische Messung mit chromogenem Substrat. Die durch humanen Faktor Xa aus dem farblosen chromogenen Substrat freigesetzte Menge an p-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die IC₅₀ berechnet als diejenige Konzentration, die den eingesetzten Faktor Xa um 50 % hemmt.

### Material

Tris(hydroxymethyl)-aminomethan-Puffer (100 mMol) und Natriumchlorid (150 mMol), pH 8.0 plus 1 mg/ml Human Albumin Fraction V, Proteasefrei.

Faktor Xa (Calbiochem), Spez. Aktivität: 217 IU/mg, Endkonzentration: 7 IU/ml pro Reaktionsansatz.

Substrat S 2765 (Chromogenix), Endkonzentration: 0.3 mM/l (1 KM) pro Reaktionsansatz.

Testsubstanz: Endkonzentration 100, 30, 10, 3, 1, 0.3, 0.1, 0.03, 0.01, 0.003, 0.001 µMol/l.

### Durchführung

10 µl einer 23.5-fach konzentrierteren Ausgangslösung der Testsubstanz bzw. Lösungsmittel (Kontrolle), 175 µl TRIS/HSA-Puffer und 25 µl Faktor Xa-Gebrauchslösung von 65.8 U/L werden 10 Minuten bei 37°C inkubiert. Nach Zugabe von 25 µl S 2765-Gebrauchslösung (2.82 mMol/L) wird die Probe im Photometer (SpectraMax 250) bei 405 nm für 600 Sekunden bei 37°C gemessen.

### Auswertung

1. Ermittlung der maximalen Zunahme (deltaOD/Minuten) über 21 Messpunkte.
2. Ermittlung der %-Hemmung bezogen auf die Lösungsmittelkontrolle.
3. Erstellen einer Dosiswirkungskurve (%-Hemmung vs Substanzkonzentration).
4. Ermittlung der IC₅₀ durch Interpolation des X-Wertes (Substanzkonzentration) der Dosiswirkungskurve bei Y = 50 % Hemmung.

Alle getesteten Verbindungen zeigen IC₅₀-Werte, die kleiner als 100 µmol/L sind.

Die erfindungsgemäß hergestellten Verbindungen sind im allgemeinen gut verträglich.

Aufgrund ihrer pharmakologischen Eigenschaften eignen sich die neuen Verbindungen und deren physiologisch verträgliche Salze zur Vorbeugung und Behandlung venöser und arterieller thrombotischer Erkrankungen, wie zum Beispiel der Vorbeugung und Behandlung von tiefen Beinvenen-Thrombosen, Thrombophlebitis, der Verhinderung von Reokklusionen nach Bypass-Operationen oder Angioplastie (PT(C)A), sowie der Okklusion bei peripheren arteriellen Erkrankungen, sowie Vorbeugung und Behandlung von Lungenembolie, der disseminierten intravaskulären Gerinnung und der schweren Sepsis, der Verhinderung und Prophylaxe der DVT in Patienten mit Exacerbation der COPD, der Behandlung der ulzerativen Colitis, der Prophylaxe und Behandlung der Koronarthrombose, der Prophylaxe des Schlaganfalls und der Verhinderung der Okklusion von Shunts.

Zusätzlich sind die erfindungsgemäßen Verbindungen zur antithrombotischen Unterstützung bei einer thrombolytischen Behandlung, wie zum Beispiel mit Alteplase, Reteplase, Tenecteplase, Staphylokinase oder Streptokinase, zur Verhinderung der Langzeitrestenose nach PT(C)A, zur Prophylaxe und Behandlung von ischämischen Vorfällen in Patienten mit allen Formen der koronaren Herzerkrankung, zur Verhinderung der Metastasierung und des Wachstums von Tumoren und von Entzündungsprozessen, z.B. bei der Behandlung der pulmonalen Fibrose, zur Prophylaxe und Behandlung der rheumatoiden Arthritis, zur Verhütung oder Verhinderung von fibrin-abhängigen Gewebsadhäsionen und/oder Narbengewebebildung sowie zur Förderung von Wundheilungsprozessen geeignet.

Die genannten Verbindungen können auch als Antikoagulantien in Zusammenhang mit der Herstellung, Lagerung, Fraktionierung oder Verwendung von Vollblut oder bei invasiven Therapieverfahren, zum Beispiel zum Beschichten von Prothesen, künstlichen Herzklappen und Kathetern zur Reduktion des Thromboserisikos eingesetzt werden.

Aufgrund ihrer pharmakologischen Eigenschaften eignen sich außerdem die neuen Verbindungen und deren physiologisch verträgliche Salze zur Behandlung der Alzheimer- und Parkinson'schen Krankheit. Eine Rationale dafür ergibt sich zum Beispiel aus folgende Befunden, aus denen man schließen kann, dass Thrombinhemmer bzw. Faktor Xa Hemmer, durch Hemmung der Thrombinbildung bzw. -aktivität, wertvolle Medikamente in der Behandlung der Alzheimer- und Parkinson'schen Krankheit darstellen könnten. Klinische und experimentelle Studien legen nahe, dass neurotoxische Mechanismen, beispielsweise die mit der Aktivierung von Proteasen der Gerinnungskaskade einhergehende Entzündung, beteiligt ist am Absterben von Neuronen infolge von Hirntraumata. Verschiedene Studien deuten auf eine Beteiligung von Thrombin bei neurodegenerativen Prozessen hin, beispielsweise infolge eines Schlaganfalls, wiederholter Bypassoperation oder traumatischen Hirnverletzungen. Eine erhöhte Thrombinaktivität konnte beispielsweise noch Tage nach peripherer Nervenverletzung nachgewiesen werden. Es konnte weiterhin gezeigt werden, dass Thrombin eine Neuritenretraktion, sowie Glia-Proliferation, und Apoptose in Primärkulturen von Neuronen und Neuroblastomzellen hervorruft (zur Übersicht siehe: Neurobiol. Aging, 2004, 25(6), 783-793). Darüberhinaus deuten verschiedene in vitro Studien an Gehirnen von Patienten mit Alzheimer-Krankheit darauf hin, dass Thrombin in der Pathogenese dieser Krankheit eine Rolle spielt (Neurosci. Lett., 1992, 146, 152-54). Eine Anreicherung immunreaktiven Thrombins konnte in Neuriten-Plaques in Gehirnen von Alzheimer-Patienten nachgewiesen werden. In vitro wurde gezeigt, dass Thrombin ebenfalls eine Rolle bei der Regulation und Stimulation der Produktion des "Amyloid Precursor Proteins" (APP) spielt sowie bei der Spaltung des APP in Fragmente, welche in den Amyloid-Plaques im Gehirn von Alzheimer-Patienten nachgewiesen werden können. Weiterhin konnte gezeigt werden, dass die thrombin-induzierte mikrogliale Aktivierung in vivo zur Degeneration von nigralen dopaminergen Neuronen führt. Diese Befunde lassen den Schluss zu, dass mikrogliale Aktivierung -ausgelöst durch endogene Substanz(en) wie beispielsweise Thrombin- beteiligt sind am neuropathologischen Prozess des Zelltodes dopaminerger Neurone, wie er bei Patienten mit Parkinson'scher Krankheit vorkommt (J. Neurosci., 2003, 23, 5877-86).

Die neuen Verbindungen und ihre physiologisch verträglichen Salze sind auch für die Prophylaxe und Behandlug von arteriellen vaskulären Erkrankungen in der Kombinationstherapie mit lipidsenkenden Wirkstoffen wie HMG-CoA Reduktase Inhibitoren und Vasodilatatoren, insbesondere ACE-Inhibitoren, Angiotensin II-Antagonisten, Renin-Inhibitoren, β-Rezeptor-Antagonisten, a-Rezeptor-Antagonisten, Diuretika, Ca-Kanalblockern, oder Stimulatoren der löslichen Guanylatzyklase einsetzbar.

Durch Erhöhung der antithrombotischen Wirkung sind die neuen Verbindungen und ihre physiologisch verträglichen Salze auch in der Kombinationstherapie mit anderen Antikoagulantien wie beispielsweise unfraktioniertem Heparin, niedermolekularem Heparin, Fondaparinux oder direkten Thrombinhemmern, zum Beispiel rekombinantem Hirudin oder "active-site"-Thrombinhemmern, einsetzbar

Die neuen Verbindungen und deren physiologisch verträgliche Salze können therapeutisch in Kombination mit Azetylsalizylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Prasugrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

Die zur Erzielung einer entsprechenden Wirkung erforderliche Dosierung beträgt zweckmäßigerweise bei intravenöser Gabe 0.01 bis 3 mg/kg, vorzugsweise 0.03 bis 1.0 mg/kg, und bei oraler Gabe 0.03 bis 30 mg/kg, vorzugsweise 0.1 bis 10 mg/kg, jeweils 1 bis 4 x täglich.

Hierzu lassen sich die erfindungsgemäß hergestellten Verbindungen der Formel (I), gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die neuen Verbindungen und deren physiologisch verträgliche Salze können therapeutisch in Kombination mit Acetylsalicylsäure, mit Inhibitoren der Plättchen-Aggregation wie Fibrinogen-Rezeptorantagonisten (z.B. Abciximab, Eptifibatide, Tirofiban, Roxifiban), mit physiologischen Aktivatoren und Inhibitoren des Gerinnungssystems und deren rekombinanter Analoga (z.B. Protein C, TFPI, Antithrombin), mit Inhibitoren der ADP-induzierten Aggregation (z.B. Clopidogrel, Ticlopidin), mit P₂T-Rezeptorantagonisten (z.B. Cangrelor) oder mit kombinierten Thromboxan Rezeptorantagonisten/Synthetaseinhibitoren (z.B. Terbogrel) eingesetzt werden.

### Experimenteller Teil

Die nachfolgenden Beispiele sollen die Erfindung näher erläutern, ohne diese jedoch in ihrem Umfang zu beschränken.

Für die hergestellten Verbindungen liegen in der Regel Schmelzpunkte und/oder IR-, UV-, ¹H-NMR und/oder Massenspektren vor. Wenn nicht anders angegeben, wurden R_{f}-Werte unter Verwendung von DC-Fertigplatten Kieselgel 60 F₂₅₄ (E. Merck, Darmstadt, Artikel-Nr. 1.05714) ohne Kammersättigung bestimmt. Die unter der Bezeichnung Alox ermittelten R_{f}-Werte wurden unter Verwendung von DC-Fertigplatten Aluminiumoxid 60 F₂₅₄ (E. Merck, Darmstadt, Artikel-Nr. 1.05713) ohne Kammersättigung bestimmt. Die unter der Bezeichnung Reversed-Phase-8 ermittelten R_{f}-Werte wurden unter Verwendung von DC-Fertigplatten RP-8 F₂₅₄ₛ (E. Merck, Darmstadt, Artikel-Nr. 1.15684) ohne Kammersättigung bestimmt. Die bei den Fliessmitteln angegebenen Verhältnisse beziehen sich auf Volumeneinheiten der jeweiligen Lösungsmittel. Zu chromatographischen Reinigungen wurde Kieselgel der Firma Millipore (MATREX^{™}, 35-70 µm) verwendet. Falls nähere Angaben zur Konfiguration fehlen, bleibt offen, ob es sich um reine Stereoisomere oder um Enantiomeren-/Diastereomerengemische handelt.

Die HPLC-MS Daten wurden unter den folgenden Bedingungen erzeugt.

### Methode A:

Waters Alliance 2695, Waters Micromass ZQ Massen Spektrometer mit Diodenarraydetektor 2996.
Als mobile Phase wurde eingesetzt:
A: Wasser mit 0.13% TFA
B: Acetonitril

| Zeit in min | %A | %B | Flussrate in ml/min |
|---|---|---|---|
| 0.00 | 95 | 5 | 6.00 |
| 0.01 | 95 | 5 | 6.00 |
| 0.89 | 2 | 98 | 6.00 |
| 0.90 | 2 | 98 | 6.00 |
| 0.95 | 95 | 5 | 6.00 |
| 1.05 | 95 | 5 | 6.00 |
| 1.10 | 95 | 5 | 0.10 |

Als stationäre Phase diente eine Säule Varian MS 100 C18, 3 µm, 4.6 mm x 30 mm.

### Methode B:

Waters Alliance 2695, Waters Micromass ZQ Massen Spektrometer mit Diodenarraydetektor 2996.
Als mobile Phase wurde eingesetzt:
A: Wasser mit 0.13% TFA
B: Acetonitril

| Zeit in min | %A | %B | Flussrate in ml/min |
|---|---|---|---|
| 0.00 | 95 | 5 | 3.50 |
| 0.18 | 95 | 5 | 3.50 |
| 2.00 | 2 | 98 | 3.50 |
| 2.20 | 2 | 98 | 3.50 |
| 2.30 | 95 | 5 | 3.50 |
| 2.50 | 95 | 5 | 3.50 |
| 2.60 | 95 | 5 | 0.10 |

Als stationäre Phase diente eine Säule Varian MS 100 C18, 3 µm, 4.6 mm x 30 mm.

Die Diodenarraydetektion erfolgte im Wellenlängenbereich 210-380 nm.

### Methode C:

Waters Alliance 2695, Waters Micromass ZQ Massen Spektrometer mit Diodenarraydetektor 2996.
Als mobile Phase wurde eingesetzt:
A: Wasser mit 0.1% Ammoniak
B: Acetonitril

| Zeit in min | %A | %B | Flussrate in ml/min |
|---|---|---|---|
| 0.00 | 95 | 5 | 4.00 |
| 0.01 | 95 | 5 | 4.00 |
| 0.89 | 2 | 98 | 4.00 |
| 0.90 | 2 | 98 | 4.00 |
| 0.95 | 95 | 5 | 4.00 |
| 1.05 | 95 | 5 | 4.00 |
| 1.10 | 95 | 5 | 0.10 |

Als stationäre Phase diente eine Säule Waters Xbridge C18, 3.5 µm, 4.6 mm x 20 mm.

Die Diodenarraydetektion erfolgte im Wellenlängenbereich 210-380 nm.

### Methode D:

Waters Alliance 2695, Waters Micromass ZQ Massen Spektrometer mit Diodenarraydetektor 2996.
Als mobile Phase wurde eingesetzt:
A: Wasser mit 0.13% Trifluoressigsäure
B: Acetonitril

| Zeit in min | %A | %B | Flussrate in ml/min |
|---|---|---|---|
| 0.00 | 100 | 0 | 5.00 |
| 0.08 | 100 | 0 | 5.00 |
| 1.70 | 0 | 100 | 5.00 |
| 1.75 | 0 | 100 | 5.00 |
| 1.80 | 100 | 0 | 5.00 |
| 1.85 | 100 | 0 | 5.00 |
| 1.90 | 100 | 0 | 0.10 |

Als stationäre Phase diente eine Varian Polaris C18, 3 µm, 4.6 mm x 30 mm.

Die Diodenarraydetektion erfolgte im Wellenlängenbereich 210-380 nm.

### Methode E:

Waters Alliance 2695, Waters Micromass ZQ Massen Spektrometer mit Diodenarraydetektor 2996.
Als mobile Phase wurde eingesetzt:
A: Wasser mit 0.1% Ammoniak
B: Acetonitril

| Zeit in min | %A | %B | Flussrate in ml/min |
|---|---|---|---|
| 0.00 | 95 | 5 | 4.00 |
| 0.01 | 95 | 5 | 4.00 |
| 0.89 | 2 | 98 | 4.00 |
| 0.90 | 2 | 98 | 4.00 |
| 0.95 | 95 | 5 | 4.00 |
| 1.05 | 95 | 5 | 4.00 |
| 1.10 | 95 | 5 | 0.50 |

Als stationäre Phase diente eine Säule Waters Xbridge C18, 3.5 µm, 4.6 mm x 20 mm.

Die Diodenarraydetektion erfolgte im Wellenlängenbereich 210-380 nm.

In den Versuchsbeschreibungen werden die folgenden Abkürzungen verwendet.
- DCM: Dichlormethan
- DIPEA: *N*-Ethyl-diisopropylamin
- DMF: *N,N*-Dimethylformamid
- EtOH: Ethanol
- ges.: gesättigt
- h: Stunde(n)
- HATU: *O*-(7-Azabenzotriazol-1-yl)-*N,N,N',N'*-tetramethyluroniumhexafluorphosphat
- i. Vak.: im Vakuum
- konz.: konzentriert
- min: Minute(n)
- NMM: N-Methyl-morpholin
- R_{f}: Retentionsfaktor
- Rₜ: Retentionszeit
- TBTU: *O*-(Benzotriazol-1-yl)-*N*,*N*,*N'*,*N'*-tetramethyluroniumtetrafluorborat
- TEA: Triethylamin
- TFA: Trifluoressigsäure
- THF: Tetrahydrofuran

### Beispiel 1

### 5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz)

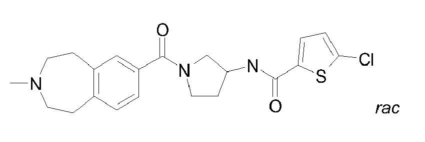

### (a) 3-[(5-Chlor-thiophen-2-carbonyl)-amino]-pyrrolidin-1-carbonsäure-tert.-butylester

0.5 g (2.7 mmol) 3-Amino-pyrrolidin-1-carbonsäure-tert.-butylester werden in 7 ml DCM gelöst, mit 1.4 ml (10.1 mmol) TEA und 0.5 g (2.7 mmol) 5-Chlorthiophen-2-carbonsäurechlorid versetzt und eine Stunde bei RT gerührt. Das Reaktionsgemisch wird mit DCM verdünnt und nacheinander mit verd. wässriger KHSO₄-Lösung, ges. wässriger NaHCO3-Lösung und Wasser gewaschen. Die vereinigten organischen Phasen werden über Natriumsulfat getrocknet und i. Vak. bis zur Trockene eingeengt.
Rₜ-Wert: 0.65 min (Methode A)
C₁₄H₁₉ClN₂O₃S (330.83)
Massenspektrum: (M+H)⁺ = 329/331 (Chlorisotope)

### (b) 5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz)

150 mg (453 mmol) 3-[(5-Chlor-thiophen-2-carbonyl)-amino]-pyrrolidin-1-carbonsäure-tert.-butylester werden in einem Gemisch aus DCM/TFA (v/v 1:1) bei RT für 30 min gerührt. Dann wird 1 ml (76 mmol) TEA zugetropft, so dass die Mischung alkalisch reagiert.

In einem weiteren Reaktionsgefäß werden 110 mg (455 mmol) 3-Methyl-2,3,4,5-tetrahydro-1H-benzo[d]azepin-7-carbonsäure-Hydrochlorid in 5 ml DCM vorgelegt und nacheinander mit 0.3 ml (2.3 mmol) TEA und 0.2 g (0.5 mmol) TBTU versetzt. Diese Reaktionsmischung wird für 20 min bei RT gerührt, dann zu der zuvor hergestellten Amin-Lösung gegeben und für 72 Stunden gerührt. Das Reaktionsgemisch wird i. Vak. eingeengt, und der Rückstand wird mit TFA sauer gestellt und mittels RP-HPLC gereinigt.
Rₜ-Wert: 1.16 min (Methode B)
C₂₁H₂₄CIN₂O₃S x CF₃CO₂H (417.96)
Massenspektrum: (M+H)⁺ = 418/420 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **2** | 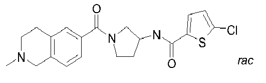 | (M+H)⁺ = 404/406 (Chlorisotope) | Rₜ-Wert = 0.44 min (Methode A) |
| | 5-Chlor-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **3** | 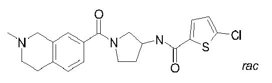 | (M+H)⁺ = 404/406 (Chlorisotope) | Rₜ-Wert = 0.44 min (Methode A) |
| | 5-Chlor-thiophen-2-carbonsäure-[1-(2-methyl-1,2,3,4-tetrahydro-isochinolin-7-carbonyl)-pyrrolidin-3-yl]-amid (als Trifluoracetat-Salz) | | |
| **4** | 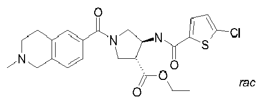 | (M+H)⁺ = 476/478 (Chlorisotope) | Rₜ-Wert = 1.25 min (Methode B) |
| | (3RS,4RS)-4-[(5-Chlor-thiophen-2-carbonyl)-amino]-1-(2-methyl-1,2,3,4-tetrahydro-isochinolin-6-carbonyl)-pyrrolidin-3-carbonsäure ethylester (als Trifluoracetat-Salz) | | |
| **5** | 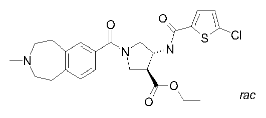 | (M+H)⁺ = 490/492 (Chlorisotope) | Rₜ-Wert: 1.29 min (Methode B) |
| | (3RS,4RS)-4[(5-Chlor-thiophen-2-carbonyl)-amino]-1-(3-methyl-2,3,4,5-tetrahydro-1H-benzo[d]azepin-7-carbonyl)-pyrrolidin-3-carbonsäure-ethylester (als Trifluoracetat-Salz) | | |
| **8** | 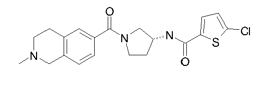 | (M+H)⁺ = 404/406 (Chlorisotope) | Rₜ-Wert = 1.14 min (Methode B) |
| | (R)-5-Chlor-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **9** | 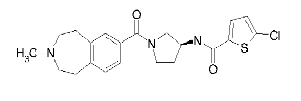 | (M+H)⁺ = 418/420 (Chlorisotope) | Rₜ-Wert = 1.14 min (Methode B) |
| | (S)-5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz) | | |
| **10** | 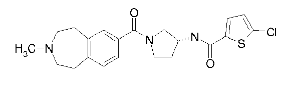 | (M+H)⁺ = 418/420 (Chlorisotope) | Rₜ-Wert = 1.17 min (Methode B) |
| | (R)-5-Chlor-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz) | | |
| **11** | 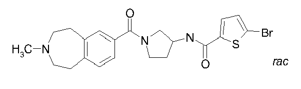 | (M+H)⁺ = 462/464 (Bromisotope) | Rₜ-Wert = 1.15 min (Methode B) |
| | 5-Brom-thiophen-2-carbonsäure-[1-(3-methyl-2,3,4,5-tetrahydro-1H-benzo[d]azepin-7-carbonyl)-pyrrolidin-3-yl]-amid (als Trifluoracetat-Salz) | | |
| **12** | 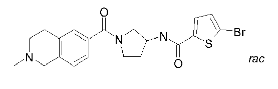 | (M+H)⁺ = 448/450 (Bromsotope) | Rₜ-Wert = 1.13 min (Methode B) |
| | 5-Brom-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **13** | 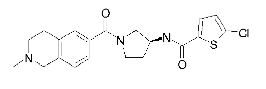 | (M+H)⁺ = 404/406 (Chlorisotope) | Rₜ-Wert = 1.14 min (Methode B) |
| | (S)-5-Chlor-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **23** | 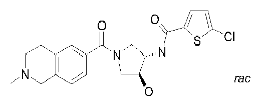 | (M+H)⁺ = 420/422 (Chlorisotope) | Rₜ-Wert = 0.99 min (Methode B) |
| | (3SR,4SR)-5-Chlor-thiophen-2-carbonsäure-[4-hydroxy-1-(2-metyl-1,2,3,4-tetrahydro-isochinolin-6-carbonyl)-pyrrolidin-3-yl]-amid (als Trifluoracetat-Salz) | | |
| **25** | 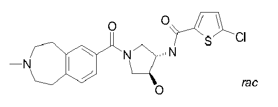 | (M+H)⁺ = 434/436 (Chlorisotope) | Rₜ-Wert: 1.02 min (Methode B) |
| | (3SR,4SR)-5-Chlor-thiophen-2-carbonsäure-[4-hydroxy-1-(3-metyl-2,3,4,5-tetrahydro-1H-benzo[d]azepin-7-carbonyl)-pyrrolidin-3-yl]-amid (als Trifluoracetat-Salz) | | |
| **28** | 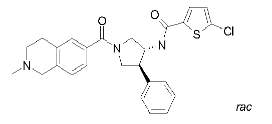 | (M+H)⁺ = 480/482 (Chlorisotope) | Rₜ-Wert = 1.32 min (Methode B) |
| | (3RS,4SR)-5-Chlor-thiophen-2-carbonsäure-[1-(2-methyl-1,2,3,4-tetrahydro-isochinolin-6-carbonyl)-4-phenyl-pyrrolidin-3-yl]-amid (als Trifluoracetat-Salz) | | |
| **29** | 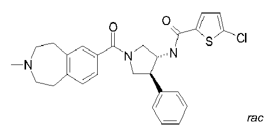 | (M+H)⁺ = 494/496 (Chlorisotope) | Rₜ-Wert: 1.36 min (Methode B) |
| | (3RS,4SR)-5-Chlor-thiophen-2-carbonsäure-[1-(3-methyl-2,3,4,5-tetrahydro-1H-benzo[d]azepin-7-carbonyl)-4-phenyl-pyrrolidin-3-yl]-amid (als Trifluoracetat-Salz) | | |

### Beispiel 6

### (3RS,4RS)-4-[(Chlor-thiophen-2-carbonyl)-amino]-1-(2-methyl-1,2,3,4-tetrahydro-isochinolin-6-carbonyl)-pyrrolidin-2-carbonsäure (als Trifluoracetat-Salz)

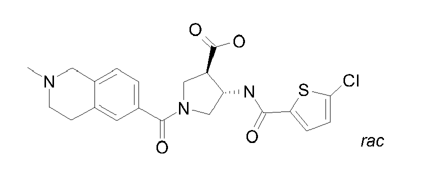

### (a) (3SR,4RS)-4-[(Chlor-thiophen-2-carbonyl)-amino]-1-(2-methyl-1,2,3,4-tetrahydro-isochinolin-6-carbonyl)-pyrrolidin-2-carbonsäure (als Trifluoracetat-Salz)

14 mg (24 µmol) (3SR,4RS)-4-[(5-Chlor-thiophen-2-carbonyl)-amino]-1-(2-methyl-1,2,3,4-tetrahydro-isochinolin-6-carbonyl)-pyrrolidin-3-carbonsäureethylester (als Trifluoracetat-Salz) werden in 500 µL Methanol gelöst, mit 120 µL Lithiumhydroxid-Lösung (8%ig in Wasser) versetzt und 16 Stunden bei RT gerührt. Man konzentriert die Mischung i.Vak. und reinigt den Rückstand mittels
RP-HPLC.
Rₜ-Wert: 1.07 min (Methode B)
C₂₁H₂₂ClN₃O₄S x CF₃CO₂H (447.94)
Massenspektrum: (M+H)⁺ = 448/450 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **7** | 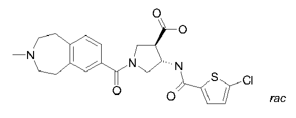 | (M+H)⁺ = 462/464 (Chlorisotope) | Rₜ-Wert: 1.10 min (Methode B) |
| | (3SR,4RS)-4-[(5-Chlor-thiophen-2-carbonyl)-amino]-1-(3-methyl-2,3,4,5-tetrahydro-1H-benzo[d]azepin-7-carbonyl)-pyrrolidin-3-carbonsäure (als Trifluoracetat-Salz) | | |
| **21** | 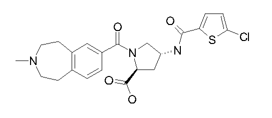 | (M+H)⁺ = 462/464 (Chlorisotope) | Rₜ-Wert: 1.11 min (Methode B) |
| | (2S,4R)-4-[(5-Chlor-thiophen-2-carbonyl)-amino]-1-(3-methyl-2,3,4,5-tetrahydro-1H-benzo[d]azepin-7-carbonyl)-pyrrolidin-2-carbonsäure (als Trifluoracetat-Salz) | | |

### Beispiel 14

### (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-methylester (als Trifluoracetat-Salz)

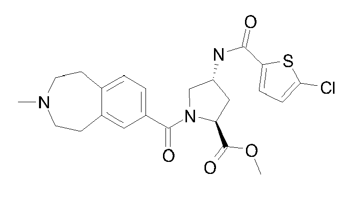

### (a) (2S,4S)-Methansulfonyloxy-pyrrolidin-1,2-dicarbonsäure-1-tert.-butylester-2-methylester

6.5 g (26.3 mmol) (2S,4S)-4-Hydroxy-pyrrolidin-1,2-dicarbonsäure-1-tert.-butylester-2-methylester werden in 40 ml DCM gelöst und bei 0°C mit 4.4 ml (31.7 mmol) TEA und 2.5 ml (32.2 mmol) Methansulfonsäurechlorid versetzt. Die Mischung wird 30 Minuten bei 0°C und zwei Stunden bei RT gerührt. Anschließend gießt man die Mischung auf Wasser und extrahiert die wässrige Phase dreimal mit DCM. Die vereinigten organischen Phasen werden über Natriumsulfat getrocknet und i.Vak. konzentriert.
Rₜ-Wert: 1.27 min (Methode B)
C₁₂H₂₁NO₇S (323.36)
Massenspektrum: (M+H)⁺ = 324

### (b) (2S,4R)-Azido-pyrrolidin-1,2-dicarbonsäure-1-tert.-butylester-2-methylester

8.5 g (26.3 mmol) (2S,4S)-Methansulfonyloxy-pyrrolidin-1,2-dicarbonsäure-1-tert.-butylester-2-methylester werden in 25 ml DMF gelöst und bei RT mit 6.0 g (92.3 mmol) Natriumazid versetzt. Die Mischung wird 20 Stunden bei 50°C gerührt. Anschließend konzentriert man das Reaktionsgemisch i.Vak. und versetzt den Rückstand mit Ethylacetat und Wasser. Die wässrige Phase wird dreimal mit Ethylacetat extrahiert. Die vereinigten organischen Phasen werden über Natriumsulfat getrocknet und i.Vak. konzentriert.
Rₜ-Wert: 1.38 min (Methode B)
C₁₁H₁₈N₄O₄ (270.29)
Massenspektrum: (M+H)⁺ = 271

### (c) (2S,4R)-Amino-pyrrolidin-1,2-dicarbonsäure-1-tert.-butylester-2-methylester

7.5 g (27.8 mmol) (2S,4R)-Azido-pyrrolidin-1,2-dicarbonsäure-1-tert.-butylester-2-methylester werden in 15 ml Methanol gelöst, mit 500 mg Palladium/Kohle (10%ig) versetzt und zwei Tage mit 3 bar Wasserstoff hydriert. Anschließend wird die Mischung filtriert und i. Vak. eingeengt.
Rₜ-Wert: 0.91 min (Methode B)
C₁₁H₂₀N₂O₄ (244.29)
Massenspektrum: (M+H)⁺ = 245

### (d) (2S,4R)-4-[(5-Chlor-thiophen-2-carbonyl)-amino]-pyrrolidin-1,2-dicarbonsäure-1-tert.-butylester-2-methylester

Herstellung analog Beispiel 1 a aus (2S,4R)-Amino-pyrrolidin-1,2-dicarbonsäure-1-tert.-butylester-2-methylester und 5-Chlor-thiophen-2-carbonylchlorid.
Rₜ-Wert: 1.56 min (Methode B)
C₁₁H₂₁ClN₂O₅S (388.87)
Massenspektrum: (M-H)- = 387/389 (Chlorisotope)

### (e) (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-methylester (als Trifluoracetat-Salz)

Herstellung analog Beispiel 1 b aus (2S,4R)-4-[(5-Chlor-thiophen-2-carbonyl)-amino]-pyrrolidin-1,2-dicarbonsäure-1-tert.-butylester-2-methylester und 3-Methyl-2,3,4,5-tetrahydro-1H-benzo[d]azepin-7-carbonsäure-Hydrochlorid.
Rₜ-Wert: 1.29 min (Methode B)
C₂₃H₂₆ClN₃O₄S x CF₃CO₂H (476.00)
Massenspektrum: (M+H)⁺ = 476/478 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **15** | 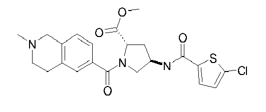 | (M+H)⁺ = 462/464 (Chlorisotope) | Rₜ-Wert: 1.01 min (Methode B) |
| | (2S,4R)-4-[(5-Chlor-thiophen-2-carbonyl)-amino]-1-(2-methyl-1,2,3,4-tetrahydro-isochinolin-6-carbonyl)-pyrrolidin-2-carbonsäure-methylester (als Trifluoracetat-Salz) | | |

### Beispiel 16

### (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäure-dimethylamid (als Trifluoracetat-Salz)

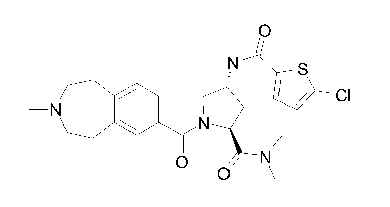

### (a) (2S,4R)-4-[(5-Chlor-thiophen-2-carbonyl)-amino]-2-dimethylcarbamoyl-pyrrolidin-1-carbonsäure-tert.-butylester

210 mg (2.6 mmol) Dimethylamin-Hydrochlorid werden in 5 ml DCM gelöst und mit 3 ml Trimethylaluminium-Lösung (2M in Toluol, 6 mmol) versetzt. Man rührt diese Mischung für 30 Minuten, gibt dann eine Lösung aus 1.0 g (2.6 mmol) (2S,4R)-4-[(5-Chlor-thiophen-2-carbonyl)-amino]-pyrrolidin-1,2-dicarbonsäure-1-tert.-butylester-2-methylester in 5 ml DCM hinzu und rührt 16 Stunden bei RT. Dann werden weitere 3 mmol Dimethylaluminium-dimethylamid-Lösung in DCM/Toluol (hergestellt analog) zugegeben und weitere drei Tage bei RT gerührt. Man verdünnt die Mischung mit 20 ml DCM und versetzt mit wenig Wasser. Diese Mischung wird i. Vak. eingeengt. Der Rückstand wird mit DCM versetzt und nacheinander mit Wasser und 0.5 N Natronlauge gewaschen. Die organische Phase wird einmal mit 0.5 N Salzsäure gewaschen, über Natriumsulfat getrocknet und i.Vak. eingeengt.
Rₜ-Wert: 1.32 min (Methode B)
C₁₇H₂₄ClN₃O₄S (401.91)
Massenspektrum: (M+H)⁺ = 402/404 (Chlorisotope)

### (b) (2S,4R)-4-[(Chlor-thiophen-2-carbonyl)-amino]-1-(3-methyl-2,3,4,5-tetrahydro-1H-benzo[d]azepin-7-carbonyl)-pyrrolidin-2-carbonsäuremethylester (als Trifluoracetat-Salz)

Herstellung analog Beispiel 1 b aus (2S,4R)-4-[(5-Chlor-thiophen-2-carbonyl)-amino]-2-dimethylcarbamoyl-pyrrolidin-1-carbonsäure-tert.-butylester und 3-Methyl-2,3,4,5-tetrahydro-1H-benzo[d]azepin-7-carbonsäure-Hydrochlorid.
Rₜ-Wert: 1.07 min (Methode B)
C₂₄H₂₉ClN₄O₃S x CF₃CO₂H (489.04)
Massenspektrum: (M+H)⁺ = 489/491 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name**. | | |
| **17** | 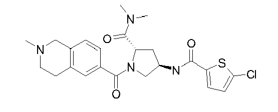 | (M+H)⁺ = 475/477 (Chlorisotope) | Rₜ-Wert: 1.05 min (Methode B) |
| | (2S,4R)-4-[(5-Chlor-thiophen-2-carbonyl)-amino]-1-(2-methyl-1,2,3,4-tetrahydro-isochinolin-6-carbonyl)-pyrrolidin-2-carbonsäure-dimethylamid (als Trifluoracetat-Salz) | | |
| **33** | 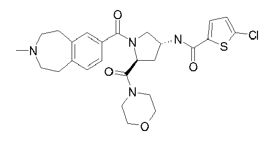 | (M+H)⁺ = 531/533 (Chlorisotope) | Rₜ-Wert: 1.09 min (Methode B) |
| | (2S,4R)-5-Chlor-thiophen-2-carbonsäure-[1-(3-methyl-2,3,4,5-tetrahydro-1H-benzo[d]azepin-7-carbonyl)-5-(morpholin-4-carbonyl)-pyrrolidin-3-yl]-amid (als Trifluoracetat-Salz) | | |
| **34** | 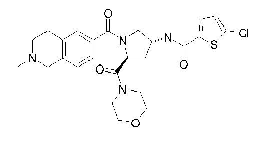 | (M+H)⁺ = 517/519 (Chlorisotope) | Rₜ-Wert: 1.05 min (Methode B) |
| | (2S,4R)-5-Chlor-thiophen-2-carbonsäure-[2-methyl-1,2,3,4-tetrahydro-isochinolin-6-carbonyl)-5-(morpholin-4-carbonyl)-pyrrolidin-3-yl]-amid (als Trifluoracetat-Salz) | | |

### Beispiel 19

### (3RS,4SR)-5-Chlor-thiophen-2-carbonsäure-[4-hydroxymethyl-1-(2-methyl-1,2,3,4-tetrahydro-isochinolin-6-carbonyl)-pyrrolidin-3-yl]-amid (als Trifluoracetat-Salz)

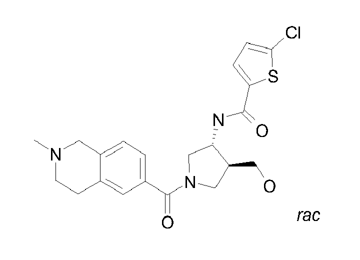

### (a) (3RS,4SR)-3-[(5-Chlor-thiophen-2-carbonyl)-amino]-4-hydroxymethyl-pyrrolidin-1-carbonsäure-tert.-butylester

168 mg (417 µmol) (3RS,4RS)-4-[(5-Chlor-thiophen-2-carbonyl)-amino]-pyrrolidin-1,3-dicarbonsäure-1-tert.-butylester-3-ethylester werden in 4 ml THF gelöst, portionsweise mit insgesamt 20 mg (872 µmol) Lithiumborhydrid versetzt und für eine Stunde bei RT gerührt. Anschließend wird die Reaktionsmischung in ges. Natriumchlorid-Lösung gegossen und gerührt. Man extrahiert die wässrige Phase dreimal mit Ethylacetat, trocknet die vereinigten organischen Phasen über Natriumsulfat und engt i.Vak. zur Trockene ein.
Rₜ-Wert: 1.36 min (Methode B)
C₁₅H₂₁ClN₂O₄S (360.86)
Massenspektrum: (M+H)⁺ = 361/363 (Chlorisotope)

### (b) (3RS,4SR)-5-Chlor-thiophen-2-carbonsäure-[4-hydroxymethyl-1-(2-methyl-1,2,3,4-tetrahydro-isochinolin-6-carbonyl)-pyrrolidin-3-yl]-amid

Herstellung analog Beispiel 1 b aus (3RS,4RS)-3-[(5-Chlor-thiophen-2-carbonyl)-amino]-4-hydroxymethyl-pyrrolidin-1-carbonsäure-tert.-butylester und 2-Methyl-1,2,3,4-tetrahydro-isochinolin-6-carbonsäure-Hydrochlorid.
Rₜ-Wert: 1.00 min (Methode B)
C₂₁H₂₄ClN₃O₃S (433.95)
Massenspektrum: (M+H)⁺ = 434/436 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **18** | 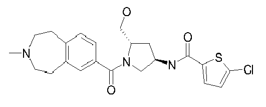 | (M+H)⁺ = 448/450 (Chlorisotope) | Rₜ-Wert: 1.03 min (Methode B) |
| | (2S,4R)-5-Chlor-thiophen-2-carbonsäure-[5-hydroxymethyl-1-(3-methyl-2,3,4,5-tetrahydro-1H-benzo[d]azepin-7-carbonyl)-pyrrolidin-3-yl]-amid | | |
| **20** | 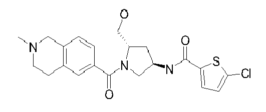 | (M+H)⁺ = 434/436 (Chlorisotope) | Rₜ-Wert: 1.00 min (Methode B) |
| | (2S,4R)-5-Chlor-thiophen-2-carbonsäure-[5-hydroxymethyl-1-(2-methyl-1,2,3,4-tetrahydro-isochinolin-6-carbonyl)-pyrrolidin-3-yl]-amid (als Trifluoracetat-Salz) | | |

### Beispiel 22

### (3RS,4RS)-5-Chlor-thiophen-2-carbonsäure-[4-methoxy-1-(2-methyl-1,2,3,4-tetrahydro-isochinolin-6-carbonyl)-pyrrolidin-3-yl]-amid (als Trifluoracetat-Salz)

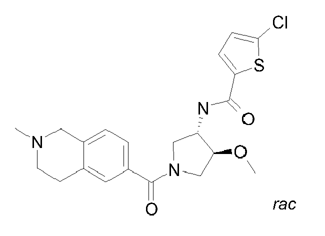

### (a) (3SR,4SR)-3-[(5-Chlor-thiophen-2-carbonyl)-amino]-4-methoxy-pyrrolidin-1-carbonsäure-tert.-butylester

Herstellung analog Beispiel 1a aus (3SR,4SR)-3-Amino-4-methoxy-pyrrolidin-1-carbonsäure-tert.-butylester (hergestellt analog Y. Tsuzuki et al. Tetrahedron Asymm. 2001, 12, 2989) und 5-Chlorthiophen-2-carbonylchlorid.
Rₜ-Wert: 1.36 min (Methode B)
C₁₅H₂₁CIN₂O₄S (360.86)
Massenspektrum: (M+H)⁺ = 361/363 (Chlorisotope)

### (b) (3SR,4SR)-5-Chlor-thiophen-2-carbonsäure-[4-methoxy-1-(2-methyl-1,2,3,4-tetrahydro-isochinolin-6-carbonyl)-pyrrolidin-3-yl]-amid (als Trifluoracetat-Salz)

Herstellung analog Beispiel 1 b aus (3SR,4SR)-3-[(5-Chlor-thiophen-2-carbonyl)-amino]-4-methoxy-pyrrolidin-1-carbonsäure-tert.-butylester und 2-Methyl-1,2,3,4-tetrahydro-isochinolin-6-carbonsäure-Hydrochlorid.
Rₜ-Wert: 1.11 min (Methode B)
C₂₁H₂₄ClN₃O₃S (433.96)
Massenspektrum: (M+H)⁺ = 434/436 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **24** | 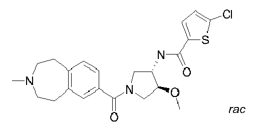 | (M+H)⁺ = 448/450 (Chlorisotope) | Rₜ-Wert: 1.12 min (Methode B) |
| | (3SR,4SR)-5-Chlor-thiophen-2-carbonsäure-[4-methoxy-1-(3-methyl-2,3,4,5-tetrahydro-1H-benzo[d]azepin-7-carbonyl)-pyrrolidin-3-yl]-amid (als Trifluoracetat-Salz) | | |

### Beispiel 26

### (2S,4R)-4-[(5-Chlor-thiophen-2-carbonyl)-amino]-1-[(3R)-2,3-dimethyl-1,2,3,4-tetrahydroisochinolin-6-carbonyl]-pyrrolidin-2-carbonsäure-dimethylamid (als Trifluoracetat-Salz)

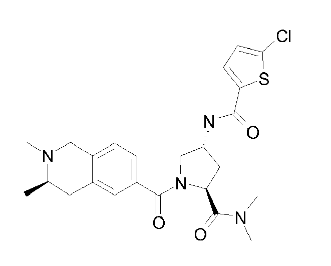

### (a) (S_{S},R)-2-Methyl-propan-2-sulfinsäure-[2-(5-brom-2-cyano-phenyl)-1-methyl-ethyl]-amid

6,0 ml (42,8 mmol) Diisopropylamin werden in 80 ml THF gelöst, langsam mit 26,7 ml (42,8 mmol) Butyllithium-Lösung (1.6 M in n-Hexan) bei 0°C versetzt, für 30 min nachgerührt. Anschließend wird diese Lösung auf -78°C abgekühlt und eine Lösung von 4,0 g (20.4 mmol) 4-Brom-2-methyl-benzonitril in 15 ml THF langsam zugetropft. Man rührt diese Mischung 70 Minuten bei -78°C und tropft dann eine Lösung von 1,5 g (10.2 mmol) (S_{S})-Ethyliden-N-tert.-butyl-sulfinamid (hergestellt analog J. Ellman et al. J. Org. Chem. 2001, 66, 8772 aus Acetaldehyd und (S_{S})-tert.-Butylsulfinamid) in 15 ml THF zu. Man rührt 2,5 Stunden bei -70 - -65°C. Die Reaktionsmischung wird mit 5 ml ges. Ammoniumchlorid-Lösung versetzt und nach dem Auftauen mit Wasser und Ethylacetat versetzt. Man extrahiert die wässrige Phase dreimal mit Ethylacetat, trocknet die vereinigten organischen Phasen über Natriumsulfat und engt i. Vak. zur Trockene ein. Der Rückstand wird säulenchromatographisch an Silicagel gereinigt (Fließmittel DCM/MeOH 100:3)
Rₜ-Wert: 1.45 min (Methode B)
C₁₄H₁₉BrN₂OS (343.28)
Massenspektrum: (M+H)⁺ = 343/345 (Bromisotope)

### (b) (R)-2-(2-Amino-propyl)-4-brom-benzonitril (als Hydrochlorid-Salz)

830 mg (2.4 mmol) (S_{S},R)-2-Methyl-propan-2-sulfinsäure-[2-(5-brom-2-cyano-phenyl)-1-methyl-ethyl]-amid werden in 10 ml ethanolischer Salzsäure (40%ig) gelöst und für 3 Stunden bei 60°C und dann 16 Stunden bei RT gerührt. Anschließend wird die Reaktionsmischung zur Trockene eingeengt.
Rₜ-Wert: 0.97 min (Methode B)
C₁₀H₁₁BrN₂ x HCl (239.12)
Massenspektrum: (M+H)⁺ = 239/241 (Bromisotope)

### (c) (R)-6-Brom-3-methyl-3,4-dihydro-2H-isochinolin-1-on

480 mg (1.7 mmol) (R)-2-(2-Amino-propyl)-4-brom-benzonitril (als Hydrochlorid-Salz) werden in 5 ml 10 N Natronlauge gelöst und für 16 Stunden bei 80°C gerührt. Anschließend wird die Reaktionsmischung mit Salzsäure sauer gestellt und dreimal mit Ethylacetat extrahiert. Die vereinigten ogranischen Phasen werden über Natriumsulfat getrocknet, filtriert und i. Vak. eingeengt. Der Rückstand wird mittels RP-HPLC gereinigt.
Rₜ-Wert: 1.31 min (Methode B)
C₁₀H₁₀BrNO (240.10)
Massenspektrum: (M+H)⁺ = 240/242 (Bromisotope)

### (d) (R)-6-Brom-2,3-dimethyl-3,4-dihydro-2H-isochinolin-1-on

426 mg (1.7 mmol) (R)-6-Brom-3-methyl-3,4-dihydro-2H-isochinolin-1-on werden in 3 ml DMF gelöst und bei 0°C mit 80 mg (2 mmol) Natriumhydrid (60%ig in Mineralöldispersion) versetzt. Nach 10 Minuten werden 122 µl (1.9 mmol) Methyliodid zugetropft, und die Mischung wird für 16 Stunden bei RT gerührt. Anschließend wird die Reaktionsmischung mit Wasser versetzt und dreimal mit Ethylacetat extrahiert. Die vereinigten organischen Phasen werden über Natriumsulfat getrocknet, filtriert und i. Vak. eingeengt. Der Rückstand wird mittels Flashchromatographie an Silicagel (Fließmittel DCM/MeOH 20:1) gereinigt.
Rₜ-Wert: 1.40 min (Methode B)
C₁₁H₁₂BrNO (254.12)
Massenspektrum: (M+H)⁺ = 254/256 (Bromisotope)

### (e) (R)-2,3-Dimethyl-1-oxo-1,2,3,4-tetrahydroisochinolin-6-carbonsäuremethylester

In einem inertisierten Autoklaven werden 100 mg (394 µmol) (R)-6-Brom-2,3-dimethyl-3,4-dihydro-2H-isochinolin-1-on in einer Mischung aus 20 ml MeOH und 5 ml DMF gelöst und mit 20 mg (89 µmol) Palladium(II)-acetat, 70 mg (86 µmol) 1,1'-Bis-(diphenylphospino)-ferrocen-dichlorpalladium(II)-Komplex mit DCM und mit 110 µl (08. mmol) TEA versetzt. Dann werden 2 bar Kohlenmonoxid aufgepresst und die Mischung für 16 Stunden geschüttelt. Anschließend wird noch zweimal die gleiche Menge Palladium(II)-acetat und 1,1'-Bis-(diphenylphospino)-ferrocen-dichlorpalladium(II)-Komplex mit DCM zugesetzt und jeweils weitere 24 Stunden bei gleicher Temperatur geschüttelt. Man lässt die Mischung abkühlen und filtriert vom Katalysatorgemisch ab. Das Filtrat wird i. Vak. eingeengt. Der so erhaltene Rückstand wird mittels Flashchromatographie an Silicagel (Fließmittel PE/EE 1:1) aufgereinigt. Die Produkt enthaltenden Fraktionen werden vereinigt und i. Vak. konzentriert. Das Rohprodukt wird mittel RP-HPLC gereinigt.
Rₜ-Wert: 1.22 min (Methode B)
C₁₃H₁₅NO₃ (233.26)
Massenspektrum: (M+H)⁺ = 234

### (f) (R)-2,3-Dimethyl-1,2,3,4-tetrahydroisochinolin-6-carbonsäuremethylester

60 mg (257 µmol) (R)-2,3-Dimethyl-1-oxo-1,2,3,4-tetrahydroisochinolin-6-carbonsäure-methylester werden unter Argon-Athmosphäre in 2 ml THF gelöst und bei RT mit 100 µl (542 µmol) Diphenylsilan versetzt. Anschließend werden 20 mg (21 µmol) Carbonylhydridotris(triphenylphosphin)rhodium(I) zugegeben und die Mischung für zwei Stunden gerührt. Man gibt weitere 50 µl Diphenylsilan und 10 mg Carbonylhydridotris(triphenylphosphin)rhodium(I) hinzu und rührt die Mischung weitere 2,5 Stunden. Anschließend wird das Reaktionsgemisch i. Vak. eingeengt und mittels Flashchromatographie an Silicagel (Fließmittel DCM/MeOH 95:5) gereinigt.
Rₜ-Wert: 0.93 min (Methode B)
C₁₃H₁₇NO₂ (219.28)
Massenspektrum: (M+H)⁺ = 220

### (g) (R)-2,3-Dimethyl-1,2,3,4-tetrahydroisochinolin-6-carbonsäure (als Hydrochlorid-Salz)

44 mg (201 µmol) (R)-2,3-Dimethyl-1,2,3,4-tetrahydroisochinolin-6-carbonsäure-methylester werden in 2 ml 6 N Salzsäure gelöst und bei 60°C einen Tag gerührt. Anschließend wird das Reaktionsgemisch i. Vak. konzentriert und lyophilisiert.
Rₜ-Wert: 0.62 min (Methode B)
C₁₂H₁₅NO₂ x HCl (205.26)
Massenspektrum: (M+H)⁺ = 206

### (h) (2S,4R)-4-[(5-Chlor-thiophen-2-carbonyl)-amino]-1-[(3R)-2,3-dimethyl-1,2,3,4-tetrahydroisochinolin-6-carbonyl]-pyrrolidin-2-carbonsäure-dimethylamid (als Trifluoracetat-Salz)

Herstellung analog Beispiel 1 b aus (2S,4R)-4-[(5-Chlor-thiophen-2-carbonyl)-amino]-2-dimethylcarbamoyl-pyrrolidin-1-carbonsäure-tert.-butylester und (R)-2,3-Dimethyl-1,2,3,4-tetrahydroisochinolin-6-carbonsäure (als Hydrochlorid-Salz) mit HATU als Kupplungsreagenz.
Rₜ-Wert: 1.10 min (Methode B)
C₂₄H₂₉CIN₄O₃S x CF₃CO₂H (489.04)
Massenspektrum: (M+H)⁺ = 489/491 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **30** | 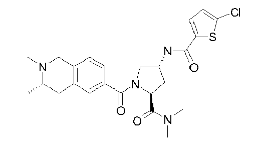 | (M+H)⁺ = 489/491 (Chlorisotope) | Rₜ-Wert: 1.16 min (Methode B) |
| | (2S,4R)-4-[(5-Chlor-thiophen-2-carbonyl)-amino]-1-[(3S)-2,3-dimethyl-1,2,3,4-tetrahydroisochinolin-6-carbonyl]-pyrrolidin-2-carbonsäure-dimethylamid (als Trifluoracetat-Salz) | | |

### Beispiel 32

### (3SR,4SR)-5-Chlor-thiophen-2-carbonsäure-[4-benzyloxy-1-(2-methyl-1,2,3,4-tetrahydro-isochinolin-6-carbonyl)-pyrrolidin-3-yl]-amid (als Trifluoracetat-Salz)

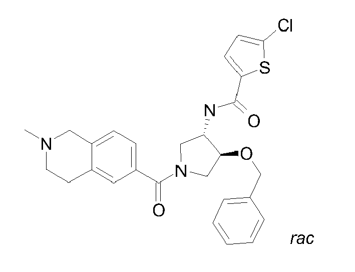

### (a) (3SR,4SR)-3-Azido-4-hydroxy-pyrrolidin-1-carbonsäure-tert.-butylester

1.7 g (9.2 mmol) rac-6-Oxa-3-aza-bicyclo[3.1.0]hexan-3-carbonsäure-tert.-butylester (hergestellt analog Y. Tsuzuki et al. Tetrahedron Asymm. 2001, 12, 2989) werden in einem Gemisch aus 16 ml 1,4-Dioxan und 3 ml Wasser gelöst, mit 1.8 g (27.5 mmol) Natriumazid versetzt und 20 Stunden bei 100°C gerührt. Anschließend wird die Reaktionsmischung abgekühlt, mit Wasser versetzt und dreimal mit Ethylacetat extrahiert. Die vereinigten organischen Phasen werden mit ges. Natriumchlorid-Lösung gewaschen und, mit Aktivkohle versetzt, über Natriumsulfat getrocknet und i. Vak. eingeengt.
R_{f}-Wert: 0.80 (Kieselgel, Fließmittel DCM/MeOH 10:1))
C₉H₁₆N₄O₃ (228.25)
Massenspektrum: (M+H)⁺ = 229

### (b) (3SR,4SR)-3-Amino-4-hydroxy-pyrrolidin-1-carbonsäure-tert.-butylester

Hergestellt analog Beispiel 14 c aus (3SR,4SR)-3-Azido-4-hydroxy-pyrrolidin-1-carbonsäure-tert.-butylester.
Rₜ-Wert: 0.42 min (Methode C)
C₉H₁₈N₂O₃ (202.25)
Massenspektrum: (M+H)⁺ = 203

### (c) (3SR,4SR)-3-[(5-Chlor-thiophen-2-carbonyl)-amino]-4-hydroxy-pyrrolidin-1-carbonsäure-tert.-butylester

201 mg (1.2 mmol) 5-Chlorthiophen-2-carbonsäure und 436 mg (1.4 mmol) TBTU werden in 5 ml DCM suspendiert und mit 260 µl (1.9 mmol) TEA versetzt. Man rührt die Mischung 30 Minuten und gibt dann eine Lösung von 250 mg (1.2 mmol) (3SR,4SR)-3-Amino-4-hydroxy-pyrrolidin-1-carbonsäure-tert.-butylester in 5 ml DCM hinzu und rührt 16 Stunden bei RT. Anschließend gießt man das Reaktionsgemisch in Wasser und extrahiert mit DCM. Die organische Phase wird mit ges. Natriumchlorid-Lösung gewaschen und, mit Aktivkohle versetzt, über Natriumsulfat getrocknet. Man filtriert und engt das Filtrat zur Trockene ein. Der so erhaltene Rückstand wird säulenchromatographisch gereinigt
(Silicagel, Fließmittel DCM/MeOH 10:1).
Rₜ-Wert: 0.70 min (Methode C)
C₁₄H₁₉C1N₂O₄S (346.83)
Massenspektrum: (M+H)⁺ = 347/349 (Chlorisotope)

### (d) (3SR,4SR)-3-Benzyloxy-4-[(5-Chlor-thiophen-2-carbonyl)-amino]-pyrrolidin-1-carbonsäure-tert.-butylester

67 mg (193 µmol) (3SR,4SR)-3-[(5-Chlor-thiophen-2-carbonyl)-amino]-4-hydroxy-pyrrolidin-1-carbonsäure-tert.-butylester werden in 500 µl DMF gelöst und nacheinander mit 15 mg (367 µmol) Natriumhydrid (60%ige Dispersion in Mineralöl) und 25 µl (212 µmol) Benzylbromid versetzt. Nach zwei Stunden werden weitere 15 mg Natriumhydrid-Dispersion zugesetzt und die Reaktionsmischung 16 Stunden bei RT gerührt. Anschließend wird das Gemisch auf Wasser gegossen und mit DCM extrahiert. Die organische Phase wird über Natriumsulfat getrocknet, filtriert und i. Vak. eingeengt. Der so erhaltene Rückstand wird säulenchromatographisch an Silicagel (Fließmittel Petrolether/Ethylacetat 3:1) gereinigt
Rₜ-Wert: 1.80 min (Methode B)
C₂₁H₂₅ClN₂O₄S (436.95
Massenspektrum: (M+H)⁺ = 437/439 (Chlorisotope)

### (e) (3SR,4SR)-5-Chlor-thiophen-2-carbonsäure-[4-benzyloxy-1-(2-methyl-1,2,3,4-tetrahydro-isochinolin-6-carbonyl)-pyrrolidin-3-yl]-amid (als Trifluoracetat-Salz)

Hergestellt analog Beispiel 1 b aus (3SR,4SR)-3-[(5-Chlor-thiophen-2-carbonyl)-amino]-4-hydroxy-pyrrolidin-1-carbonsäure-tert.-butylester.
Rₜ-Wert: 1.42 min (Methode B)
C₂₇H₂₈ClN₃O₃S (510.06)
Massenspektrum: (M+H)⁺ = 510/512 (Chlorisotope)

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **27** | 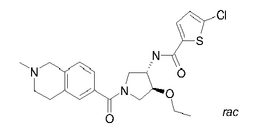 | (M+H)⁺ = 448/450 (Chlorisotope) | Rₜ-Wert: 1.21 min (Methode B) |
| | (3SR,4SR)-5-Chlor-thiophen-2-carbonsäure-[4-ethoxy-1-(3-methyl-1,2,3,4-tetrahydro-isochinolin-6-carbonyl)-pyrrolidin-3-yl]-amid (als | | |
| **31** | 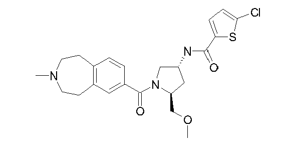 | (M+H)⁺ = 462/464 (Chlorisotope) | Rₜ-Wert: 1.16 min (Methode B) |
| | (2S,4R)-5-Chlor-thiophen-2-carbonsäure-[5-methoxymethyl-1-(3-methyl-2,3,4,5-tetrahydro-1H-benzo[d]azepin-7-carbonyl)-pyrrolidin-3-yl]-amid (als Trifluoracetat-Salz) | | |

### Beispiel 39

### (R)-5-Ethinyl-thiophen-2-carbonsäure-[1-(2-methyl-1,2,3,4-tetrahydroisochinolin-6-carbonyl)-pyrrolidin-3-yl]-amid (als Trifluoracetat-Salz)

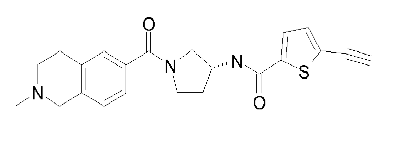

### (a) (R)-[1-(2-Methyl-1,2,3,4-tetrahydroisochinolin-6-carbonyl)-pyrrolidin-3-yl]-carbaminsäure-tert.-butylester

1.1 g (4.8 mmol) 2-Methyl-1,2,3,4-tetrahydroisochinolin-6-carbonsäure (als Hydrochlorid) werden in 12 ml DMF gelöst, mit 2.1 ml (19.3 mmol) NMM und 1.8 g (4.8 mmol) HATU versetzt und fünf Minuten bei RT gerührt. Dann werden 0.9 g (4.8 mmol) (R)-Pyrrolidin-3-yl-carbaminsäure-tert.-butylester zugegeben und die Mischung für 16 Stunden gerührt. Anschließend wird das Gemisch auf Wasser gegossen und mit DCM extrahiert. Man trennt die organische Phase über eine Phasentrennkartusche ab und engt i. Vak. zur Trockene ein. Der Rückstand wird mittels Flashchromatographie an Silicagel (Fließmittel DCM/MeOH 9:1 bis 8:2) gereinigt.
Rₜ-Wert: 1.00 min (Methode B)
C₂₀H₂₉N₃O₃ (330.83)
Massenspektrum: (M+H)⁺ = 360

### (b) (R)-(3-Amino-pyrrolidin-1-yl)-(2-methyl-1,2,3,4-tetrahydroisochinolin-6-yl)-methanon

1.4 g (3.8 mmol) (R)-[1-(2-Methyl-1,2,3,4-tetrahydroisochinolin-6-carbonyl)-pyrrolidin-3-yl]-carbaminsäure-tert.-butylester werden in 5 ml THF gelöst und langsam mit 9.5 ml Salzsäure (4 M in 1,4-Dioxan) versetzt. Man rührt für zwei Stunden, engt dann die Mischung auf 2/3 des Volumens ein und filtriert das Rohprodukt als Niederschlag ab, das anschießend mittels RP-HPLC (Fließmittel: Gradient Ammoniak/Acetonitril) gereinigt wird.
Rₜ-Wert: 0.42 min (Methode C)
C₁₅H₂₁N₃O (259.35)
Massenspektrum: (M+H)⁺ = 260

### (c) (R)-5-Ethinyl-thiophen-2-carbonsäure-[1-(2-methyl-1,2,3,4-tetrahydroisochinolin-6-carbonyl)-pyrrolidin-3-yl]-amid (als Trifluoracetat-Salz)

Hergestellt analog Beispiel 39 a aus (R)-(3-Amino-pyrrolidin-1-yl)-(2-methyl-1,2,3,4-tetrahydroisochinolin-6-yl)-methanon und 5-Ethinyl-thiophen-2-carbonsäure.
Rₜ-Wert: 1.06 min (Methode B)
C₂₂H₂₃N₃O₂S x CF₃CO₂H (393.51)
Massenspektrum: (M+H)⁺ = 394

Analog können die folgenden Verbindungen hergestellt werden:

| **Nr.** | **Strukturformel** | **Massenpeak(s)** | **R_{f}-Wert bzw. Rₜ** |
|---|---|---|---|
| | **Name** | | |
| **36** | 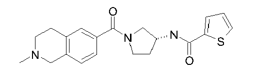 | (M+H)⁺ = 370 | Rₜ-Wert = 0.91 min (Methode B) |
| | (R)-Thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **37** | 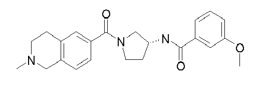 | (M+H)⁺ = 394 | Rₜ-Wert = 0.97 min (Methode B) |
| | (R)-3-Methoxy-N-[1-(2-methyl-1,2,3,4-tetrahydroisochinolin-6-carbonyl)-pyrrolidin-3-yl]-benzamid (als Trifluoracetat-Salz) | | |
| **38** | 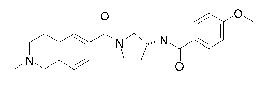 | (M+H)⁺ = 394 | Rₜ-Wert = 0.96 min (Methode B) |
| | (R)-4-Methoxy-N-[1-(2-methyl-1,2,3,4-tetrahydroisochinolin-6-carbonyl)-pyrrolidin-3-yl]-benzamid (als Trifluoracetat-Salz) | | |
| **40** | 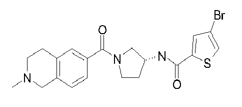 | (M+H)⁺ = 448/450 (Bromisotope) | Rₜ-Wert = 1.08 min (Methode B) |
| | (R)-4-Brom-thiophen-2-carbonsäure-[1-(2-methyl-1,2,3,4-tetrahydro-isochinolin-6-carbonyl)-pyrrolidin-3-yl]-amid (als Trifluoracetat-Salz) | | |
| **41** | 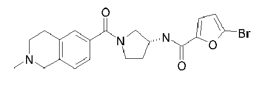 | (M+H)⁺ = 432/434 (Bromisotope) | Rₜ-Wert = 0.96 min (Methode B) |
| | (R)-5-Brom-furan-2-carbonsäure-[1-(2-methyl-1,2,3,4-tetrahydro-isochinolin-6-carbonyl)-pyrrolidin-3-yl]-amid (als Trifluoracetat-Salz) | | |
| **42** | 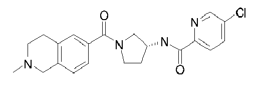 | (M+H)⁺ = 399/401 (Chlorisotope) | Rₜ-Wert = 1.01 min (Methode B) |
| | (R)-5-Chlor-pyridin-2-carbonsäure-[1-(2-methyl-1,2,3,4-tetrahydroisochinolin-6-carbonyl)-pyrrolidin-3-yl]-amid (als Bis-Trifluoracetat-Salz) | | |
| **43** | 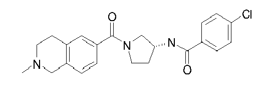 | (M+H)⁺ = 398/400 (Chlorisotope) | Rₜ-Wert = 1.01 min (Methode B) |
| | (R)-4-Chlor-N-[1-(2-methyl-1,2,3,4-tetrahydroisochinolin-6-carbonyl)-pyrrolidin-3-yl]-benzamid (als Trifluoracetat-Salz) | | |
| **44** | 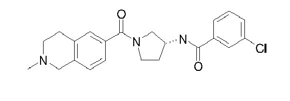 | (M+H)⁺ = 398/400 (Chlorisotope) | Rₜ-Wert = 1.01 min (Methode B) |
| | (R)-3-Chlor-N-[1-(2-methyl-1,2,3,4-tetrahydroisochinolin-6-carbonyl)-pyrrolidin-3-yl]-benzamid (als Trifluoracetat-Salz) | | |

### Beispiel 45

### (3SR,4SR)-5-Chlor-thiophen-2-carbonsäure-[4-methyl-1-(2-methyl-1,2,3,4-tetrahydroisochinolin-6-carbonyl)-pyrrolidin-3-yl]-amid (als Trifluoracetat-Salz)

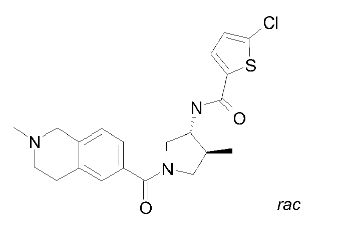

### (a) (3RS,4RS)-1-Benzyl-4-methyl-pyrrolidin-3-carbonsäure-methylester (als Trifluoracetat-Salz)

830 µl (7.8 mmol) Crotonsäuremethylester werden in 25 ml DCM gelöst und mit 61 µl (0.79 mmol) TFA versetzt. Dann wird eine Lösung aus 2,0 ml (7.8 mmol) N-Methoxymethyl-N-trimethylsilylmethyl-benzylamin in 5 ml DCM innerhalb von 20 Minuten zugetropft. Die Reaktionsmischung wird 16 Stunden gerührt und dann i. Vak. eingeengt. Der Rückstand wird mittels RP-HPLC gereinigt.
Rₜ-Wert: 0.94 min (Methode B)
C₁₄H₁₉NO₂ (233.31)
Massenspektrum: (M+H)⁺ = 234

### (b) (3RS,4RS)-1-Benzyl-4-methyl-pyrrolidin-3-carbonsäure (als Hydrochlorid-Salz)

2.0 g (5.7 mmol) (3RS,4RS)-1-Benzyl-4-methyl-pyrrolidin-3-carbonsäuremethylester (als Trifluoracetat-Salz) werden in 4 ml Methanol gelöst und mit 5 ml Lithiumhydroxid-Lösung (8%ig in Wasser) versetzt. Die Mischung wird fünf Stunden bei RT gerührt, dann mit 3.2 ml 4N Salzsäure versetzt und zur Trockene eingeengt.
Rₜ-Wert: 0.83 min (Methode B)
C₁₃H₁₇NO₂ (219.29)
Massenspektrum: (M+H)⁺ = 220

### (c) (3RS,4RS)-1-Benzyl-4-methyl-pyrrolidin-3-carbonsäure-methylester

1.3 g (4.9 mmol) (3RS,4RS)-1-Benzyl-4-methyl-pyrrolidin-3-carbonsäure (als Hydrochlorid-Salz) werden in 10 ml Methanol gelöst und unter Eisbad-Kühlung mit 0.6 ml (8.5 mmol) Thionylchlorid versetzt. Das Eisbad wird entfernt und die Mischung für drei Stunden unter Rückfluß erhitzt. Anschließend wird die Mischung i. Vak. eingeengt und mit 1 N Natronlauge versetzt. Die wässrige Phase wird dreimal mit Ethylacetat extrahiert. Die vereinigten organischen Phasen werden über Natriumsulfat getrocknet, filtriert und i. Vak. eingeengt.
Rₜ-Wert: 1.00 min (Methode B)
C₁₄H₁₉NO₂ (233.31)
Massenspektrum: (M+H)⁺ = 234

### (d) (3RS,4RS)-4-Methyl-pyrrolidin-3-carbonsäure-methylester (als Hydrochlorid-Salz)

927 mg (4 mmol) (3RS,4RS)-1-Benzyl-4-methyl-pyrrolidin-3-carbonsäuremethylester werden in 15 ml Methanol gelöst, mit 100 mg Palladium/Kohle (10%ig) und 2 ml 2N Salzsäure versetzt und 14 Stunden mit 3 bar Wasserstoff hydriert. Anschließend wird die Mischung filtriert und i. Vak. eingeengt.
Rₜ-Wert: 0.39 min (Methode B)
C₇H₁₃NO₂ x HCl (143.19)
Massenspektrum: (M+H)⁺ = 144

### (e) (3RS,4RS)-4-Methyl-1-(2-methyl-1,2,3,4-tetrahydroisochinolin-6-carbonyl)-pyrrolidin-3-carbonsäure (als Trifluoracetat-Salz)

880 mg (3.9 mmol) 2-Methyl-1,2,3,4-tetrahydroisochinolin-6-carbonsäure (als Hydrochlorid-Salz) werden in 2 ml DMF gelöst und mit 1,5 g (3.9 mmol) HATU und 1,7 ml (15.6 mmol) NMM versetzt. Man rührt fünf Minuten bei RT und gibt dann eine Lösung von 700 mg (3.9 mmol) (3RS,4RS)-4-Methyl-pyrrolidin-3-carbonsäure-methylester (als Hydrochlorid-Salz) in 2 ml DMF zu. Die Reaktionsmischung wird 16 Stunden bei RT gerührt, dann mit 2N Natronlauge versetzt und dreimal mit Ethylacetat ausgeschüttelt. Die wässrige Phase wird mit TFA sauer gestellt und mittels RP-HPLC gereinigt. Man erhält die Carbonsäure als Produkt.
Rₜ-Wert: 0.74 min (Methode B)
C₁₇H₂₂N₂O₃ x CF₃CO₂H (302.38)
Massenspektrum: (M+H)⁺ = 303

### (f) (3RS,4SR)-(3-Amino-4-methyl-pyrrolidin-1-yl)-(2-methyl-1,2,3,4-tetrahydroisochinolin-6-yl)-methanon

72 mg (173 µmol) (3RS,4RS)-4-Methyl-1-(2-methyl-1,2,3,4-tetrahydroisochinolin-6-carbonyl)-pyrrolidin-3-carbonsäure (als Trifluoracetat-Salz) werden in 4 ml tert.-Butanol gelöst und mit 95 µl (682 µmol) TEA versetzt. Dann gibt man 150 µl (675 µmol) Diphenylphosphorsäureazid hinzu und rührt für zwei Stunden unter Rückfluß. Die Reaktionsmischung wird dann mit 2N Natronlauge versetzt und dreimal mit Ethylacetat ausgeschüttelt. Die wässrige Phase wird i. Vak. auf 6 ml Volumen konzentriert und mittels RP-HPLC (Fließmittel: Ammoniak/Acetonitril) gereinigt. Man erhält das Amin als Produkt.
Rₜ-Wert: 0.46 min (Methode C)
C₁₆H₂₃N₃O (273.37)
Massenspektrum: (M+H)⁺ = 274

### (g) (3SR,4SR)-5-Chlor-thiophen-2-carbonsäure-[4-methyl-1-(2-methyl-1,2,3,4-tetrahydroisochinolin-6-carbonyl)-pyrrolidin-3-yl]-amid (als Trifluoracetat-Salz)

Hergestellt analog Beispiel 1 a aus (3RS,4RS)-(3-Amino-4-methyl-pyrrolidin-1-yl)-(2-methyl-1,2,3,4-tetrahydroisochinolin-6-yl)-methanon.
Rₜ-Wert: 1.18 min (Methode B)
C₂₁H₂₄CIN₃O₂S x CF₃CO₂H (417.96)
Massenspektrum: (M+H)⁺ = 418/420 (Chlorisotope)

### Beispiel 46

### (2S,4R)-4-[(5-Chlor-thiophen-2-carbonyl)-amino]-1-(2-methyl-2,3-dihydro-1H-isoindol-5-carbonyl)-pyrrolidin-2-carbonsäure-methylester (als Trifluoracetat-Salz)

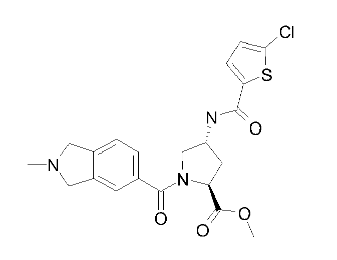

### (a) 2,3-Dihydro-1H-isoindol-5-carbonsäure-methylester

Herstellung analog Beispiel 45 c aus 2,3-Dihydro-1H-isoindol-5-carbonsäure (als Hydrochlorid-Salz; hergestellt analog EP 0 528 369).
Rₜ-Wert: 0.49 min (Methode D)
C₁₀H₁₁NO₂ (177.20)
Massenspektrum: (M+H)⁺ = 178

### (b) 2-Methyl-2,3-dihydro-1H-isoindol-5-carbonsäure-methylester

1,2 g (6.6 mmol) 2,3-Dihydro-1H-isoindol-5-carbonsäure-methylester werden in 5 ml Ameisensäure gelöst, mit 2 ml Formalin-Lösung (37%ige Lösung in Wasser) versetzt, für 3,5 Stunden auf 70°C erhitzt und für 16 Stunden bei RT gerührt. Das Reaktionsgemisch wird i. Vak. eingeengt und mit 0.1 N Natronlauge versetzt und dreimal mit Ethylacetat ausgeschüttelt. Die vereinigten organischen Phasen werden über Natriumsulfat getrocknet, filtriert und i. Vak. eingeengt.
Rₜ-Wert: 0.60 min (Methode E)
C₁₁H₁₃NO₂ (191.23)
Massenspektrum: (M+H)⁺ = 192

### (c) 2-Methyl-2,3-dihydro-1H-isoindol-5-carbonsäure (als Hydrochlorid-Salz)

Herstellung analog Beispiel 26 g aus 2-Methyl-2,3-dihydro-1H-isoindol-5-carbonsäure-methylester.
Rₜ-Wert: 0.25 min (Methode B)
C₁₀H₁₁NO₂ x HCl (177.20)
Massenspektrum: (M+H)⁺ = 178

### (d) (2S,4R)-4-[(5-Chlor-thiophen-2-carbonyl)-amino]-1-(2-methyl-2,3-dihydro-1H-isoindol-5-carbonyl)-pyrrolidin-2-carbonsäure-methylester (als Trifluoracetat-Salz)

Herstellung analog Beispiel 1 b aus (2S,4R)-4-[(5-Chlor-thiophen-2-carbonyl)-amino]-pyrrolidin-1,2-dicarbonsäure-1-tert.-butylester-2-methylester und 2-Methyl-2,3-dihydro-1H-isoindol-5-carbonsäure (als Hydrochlorid-Salz).
Rₜ-Wert: 1.14 min (Methode B)
C₂₁H₂₂CIN₃O₄S x CF₃CO₂H (447.94)
Massenspektrum: (M+H)⁺ = 448/450 (Chlorisotope)

### Beispiel 35

### (R)-5-Chlor-thiophen-2-carbonsäure-[1-(6-methyl-5,6,7,8-tetrahydro-4H-thieno[2,3-d]azepin-2-carbonyl)-pyrrolidin-3-yl]-amid (als Trifluoracetat-Salz)

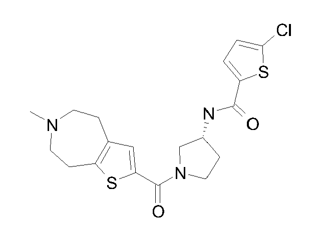

### (a) 5,6,7,8-Tetrahydro-4H-thieno[2,3-d]azepin-2-carbonsäure-methylester (als Hydrochlorid)

Herstellung analog DE 3105858 aus 6-(3-Chlor-benzyl)-5,6,7,8-tetrahydro-4H-thieno[2,3-d]azepin-2-carbonsäure-methylester.
Rₜ-Wert: 0.85 min (Methode B)
C₁₀H₁₃NO₂S x HCl (211.28)
Massenspektrum: (M+H)⁺ = 212

### (b) 6-Methyl-5,6,7,8-Tetrahydro-4H-thieno[2,3-d]azepin-2-carbonsäuremethylester

Herstellung analog Beispiel 46 b aus 5,6,7,8-Tetrahydro-4H-thieno[2,3-d]azepin-2-carbonsäure-methylester (als Hydrochlorid).
Rₜ-Wert: 0.64 min (Methode E)
C₁₁H₁₅NO₂S (225.31)
Massenspektrum: (M+H)⁺ = 226

### (c) 6-Methyl-5,6,7,8-Tetrahydro-4H-thieno[2,3-d]azepin-2-carbonsäure (als Hydrochlorid)

Herstellung analog Beispiel 45 b aus 5,6,7,8-Tetrahydro-4H-thieno[2,3-d]azepin-2-carbonsäure-methylester (als Hydrochlorid).
Ausbeute: quantitativ
C₁₀H₁₃NO₂S x HCl (211.28)
Massenspektrum: (M+H)⁺ = 212

### (d) (R)-5-Chlor-thiophen-2-carbonsäure-[1-(6-methyl-5,6,7,8-tetrahydro-4H-thieno[2,3-d]azepin-2-carbonyl)-pyrrolidin-3-yl]-amid (als Trifluoracetat-Salz)

Herstellung analog Beispiel 1 b aus(R)-3-[(5-Chlor-thiophen-2-carbonyl)-amino]-pyrrolidin-1-carbonsäure-tert.-butylester und 6-Methyl-5,6,7,8-Tetrahydro-4H-thieno[2,3-d]azepin-2-carbonsäure (als Hydrochlorid).
Rₜ-Wert: 1.15 min (Methode B)
C₁₉H₂₂ClN₃O₂S₂ x CF₃CO₂H (423.99)
Massenspektrum: (M+H)⁺ = 424/426 (Chlorisotope)

### Beispiel 47

### (3SR,4SR)-5-Chlor-thiophen-2-carbonsäure-[4-methoxy-1-(5-methyl-4,5,6,7-tetrahydro-thieno[3,2-c]pyridin-2-carbonyl)-pyrrolidin-3-yl]-amid (als Trifluoracetat-Salz)

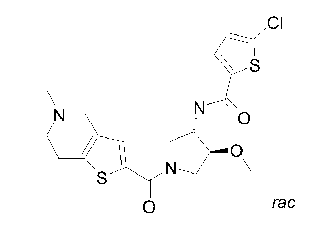

### (a) 6,7-Dihydro-4H-thieno[3,2-c]pyridin-2,5-dicarbonsäure-5-tert.-butylester-2-ethylester

1.5 ml (20.1 mmol) DMF bei 0°C langsam mit 1.5 ml (16.1 mmol) Phosphoroxychlorid versetzt. Anschließend nimmt man die Mischung in 10 ml DCM auf und rührt für 45 Minuten bei RT. Dann werden 2.2 g (9.8 mmol) 4-Oxo-piperidin-1-carbonsäure-tert.-butylester, gelöst in 10 ml DCM, zur Mischung bei 0-5°C zugetropft. Es werden weitere 10 ml DCM zugegeben und für eine Stunde bei RT gerührt. Die Reaktionsmischung wird dann auf ein Gemisch aus Eis und 20 ml gesättiger Natriumacetat-Lösung gegossen und für eine Stunde gerührt. Die organische Phase wird abgetrennt, mehrfach mit Wasser gewaschen und dann über Natriumsulfat getrocknet und i. Vak. eingeengt.
Das so erhaltene Rohprodukt wird in 15 ml DCM gelöst und mit einem Gemisch aus 1.8 ml (16.0 mmol) Mercaptoessigsäure-ethylester und 2.8 ml (19.9 mmol) TEA in 5 ml DCM versetzt. Anschließend wird das Reaktionsgemisch für 2,5 Stunden unter Rückfluß erhitzt und dann eine Stunde bei RT nachgerührt. Man gibt Wasser zu, trennt die organische Phase ab und wäscht mit viel Wasser nach. Die organische Phase wird über Natriumsulfat getrocknet, filtriert und i. Vak. eingeengt. Der Rückstand wird mittels Flashchromatographie an Silicagel (Fließmittelgemisch Cyclohexan/Ethylacetat 9:1 bis 8:2) gereinigt.
Rₜ-Wert: 1.71 min (Methode B)
C₁₅H₂₁NO₄S (311.40)
Massenspektrum: (M+H)⁺ = 312

### (b) 5-Methyl-4,5,6,7-tetrahydro-thieno[3,2-c]pyridin-2-carbonsäure-ethylester (als Trifluoracetat-Salz)

615 mg (987 µmol, 50%ige Reinheit) 6,7-Dihydro-4H-thieno[3,2-c]pyridin-2,5-dicarbonsäure-5-tert.-butylester-2-ethylester werden in 4 ml eines Gemisches aus TFA und DCM (v/v 1:1) gelöst und für 30 Minuten bei RT gerührt. Man neutralisiert das Reaktionsgemisch unter Zugabe von TEA und engt i. Vak. ein. Das so erhaltene Rohprodukt wird in 4 ml Ameisensäure gelöst und mit 0.5 ml (6.7 mmol) Formalin-Lösung (37%ig in Wasser) versetzt. Die Reaktionsmischung wird für 16 Stunden bei 70°C gerührt. Nach Abkühlen auf RT wird das Gemisch mit 50%iger wässriger Natronlauge und gesättigter Natriumhydrogencarbonat-Lösung basisch gestellt und mit Ethylacetat extrahiert. Die organische Phase wird dreimal mit Wasser gewaschen, über Natriumsulfat getrocknet, filtriert und i. Vak. eingeengt. Der Rückstand wird mittel RP-HPLC gereinigt.
Rₜ-Wert: 0.90 min (Methode B)
C₁₁H₁₅NO₂S x CF₃CO₂H (225.31)
Massenspektrum: (M+H)⁺ = 226

### (c) 5-Methyl-4,5,6,7-tetrahydro-thieno[3,2-c]pyridin-2-carbonsäure (als Hydrochlorid-Salz)

Herstellung analog Beispiel 45 b aus 5-Methyl-4,5,6,7-tetrahydro-thieno[3,2-c]pyridin-2-carbonsäure-ethylester (als Trifluoracetat-Salz).
Rₜ-Wert: 0.29 min (Methode B)
C₉H₁₁NO₂S x HCl (197.26)
Massenspektrum: (M+H)⁺ = 198

### (d) (3SR,4SR)-5-Chlor-thiophen-2-carbonsäure-[4-methoxy-1-(5-methyl-4,5,6,7-tetrahydro-thieno[3,2-c]pyridin-2-carbonyl)-pyrrolidin-3-yl]-amid (als Trifluoracetat-Salz)

Herstellung analog Beispiel 1 b aus (3SR,4SR)-3-[(5-Chlor-thiophen-2-carbonyl)-amino]-4-methoxy-pyrrolidin-1-carbonsäure-tert.-butylester und 5-Methyl-4,5,6,7-tetrahydro-thieno[3,2-c]pyridin-2-carbonsäure (als Hydrochlorid-Salz).
Rₜ-Wert: 1.17 min (Methode B)
C₁₉H₂₂ClN₃O₃S₂ x CF₃CO₂H (439.99)
Massenspektrum: (M+H)⁺ = 440/442 (Chlorisotope)

### Beispiel 48

### (3SR,4SR)-5-Chlor-thiophen-2-carbonsäure-[4-methoxy-1-(6-methyl-4,5,6,7-tetrahydro-thieno[2,3-c]pyridin-2-carbonyl)-pyrrolidin-3-yl]-amid (als Trifluoracetat-Salz)

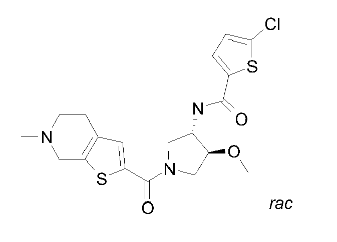

### (a) 6-Methyl-4,5,6,7-tetrahydro-thieno[2,3-c]pyridin-2-carbonsäuremethylester (als Trifluoracetat-Salz)

496 mg (2.1 mmol) 2-Brom-6-methyl-4,5,6,7-tetrahydro-thieno[2,3-c]pyridin (hergestellt analog EP 0314154 aus 6-Methyl-4,5,6,7-tetrahydro-thieno[2,3-c]pyridin) werden in 10 ml THF gelöst und auf-78°C gekühlt. Dann werden 2 ml (3.2 mmol) n-Butyllithium-Lösung (1.6 M in n-Hexan) langsam zugetropft. Das Reaktionsgemisch wird 30 Minuten bei -78°C gerührt und dann mit 1.0 ml (12.9 mmol) Chlorameisensäure-methylester versetzt. Man rührt fünf Minuten bei - 78°C, erwärmt auf RT und engt die Mischung i. Vak. ein. Der Rückstand wird mittels RP-HPLC gereinigt.
Rₜ-Wert: 0.79 min (Methode B)
C₁₀H₁₃NO₂S x CF₃CO₂H (211.28)
Massenspektrum: (M+H)⁺ = 212

### (b) 6-Methyl-4,5,6,7-tetrahydro-thieno[2,3-c]pyridin-2-carbonsäure (als Hydrochlorid-Salz)

Herstellung analog Beispiel 45 b aus 6-Methyl-4,5,6,7-tetrahydro-thieno[2,3-c]pyridin-2-carbonsäure-methylester (als Trifluoracetat-Salz).
Rₜ-Wert: 0.22 min (Methode B)
C₉H₁₁NO₂S x HCl (197.26)
Massenspektrum: (M+H)⁺ = 198

### (c) (3SR,4SR)-5-Chlor-thiophen-2-carbonsäure-[4-methoxy-1-(6-methyl-4,5,6,7-tetrahydro-thieno[2,3-c]pyridin-2-carbonyl)-pyrrolidin-3-yl]-amid (als Trifluoracetat-Salz)

Herstellung analog Beispiel 1 b aus (3SR,4SR)-3-[(5-Chlor-thiophen-2-carbonyl)-amino]-4-methoxy-pyrrolidin-1-carbonsäure-tert.-butylester und 6-Methyl-4,5,6,7-tetrahydro-thieno[2,3-c]pyridin-2-carbonsäure (als Hydrochlorid-Salz).
Rₜ-Wert: 1.16 min (Methode B)
C₁₉H₂₂ClN₃O₃S₂ x CF₃CO₂H (439.99)
Massenspektrum: (M+H)⁺ = 440/442 (Chlorisotope)

Die nachfolgenden Beispiele beschreiben die Herstellung pharmazeutischer Anwendungsformen, die als Wirkstoff eine beliebige Verbindung der allgemeinen Formel I enthalten.

### Beispiel A

### Trockenampulle mit 75 mg Wirkstoff pro 10 ml

### Zusammensetzung:

| | |
|---|---|
| Wirkstoff | 75.0 mg |
| Mannitol | 50.0 mg |
| Wasser für Injektionszwecke | ad 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel B

### Trockenampulle mit 35 mg Wirkstoff pro 2 ml

### Zusammensetzung:

| | |
|---|---|
| Wirkstoff | 35.0 mg |
| Mannitol | 100.0 mg |
| Wasser für Injektionszwecke | ad 2.0 ml |

### Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

### Beispiel C

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 9 mm.

### Beispiel D

### Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wässrigen Lösung von (4) granuliert. Dem getrockneten Granulat wird (5) zugemischt. Aus dieser Mischung werden Tabletten gepresst, biplan mit beidseitiger Facette und einseitiger Teilkerbe.
Durchmesser der Tabletten: 12 mm.

### Beispiel E

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 3 abgefüllt.

### Beispiel F

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in HartgelatineSteckkapseln Größe 0 abgefüllt.

### Beispiel G

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Verbindungen der allgemeinen Formel (I) 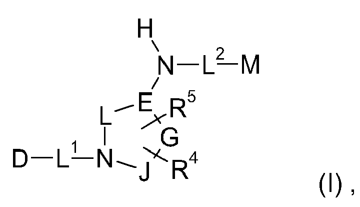 in denen
D ein substituiertes bicyclisches Ringsystem der Formel (IIa), (IIb) oder (IIc) 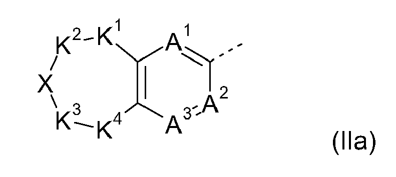 oder 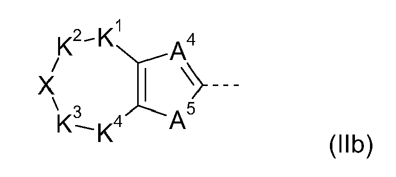 oder 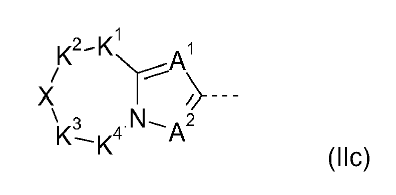 darstellt, in dem
K¹ und K⁴ jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c}
jeweils unabhängig voneinander ein Fluoratom, eine Hydroxy-, C₁₋₅-Alkyloxy-, Amino-, C₁₋₅-Alkylamino-, Di-(C₁₋₅-alkyl)-amino-, C₃₋₅-Cycloalkylenimino-, C₁₋₅-Alkylcarbonylaminogruppe,
eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkylgruppe bedeutet,
wobei nicht gleichzeitig beide Reste R^{7b}/R^{7c} über ein Heteroatom an das Ringkohlenstoffatom gebunden sein können, außer -C(R^{7b}R^{7c})- entspricht einer -CF₂-Gruppe, oder
R^{7a} eine durch Fluor-, Chlor- , Brom-, Methyl-, Methoxy-, Amino- oder Nitro-substituierte Phenyl- oder monocyclische Heteroarylgruppe bedeutet, oder
zwei Reste R^{7b}/R^{7c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, 1,3-Dioxolan-, 1,4-Dioxan-, Hexahydropyridazin-, Piperazin-, Thiomorpholin-, Morpholin-, 2-Imidazolidinon-, 2-Oxazolidinon-, Tetrahydro-2(1H)-pyrimidinon- oder [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem N-Atom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder bei dem das Schwefelatom zu einer Sulfoxid oder Sulfongruppe oxidiert sein kann,
K² und K³
jeweils unabhängig voneinander eine -CH₂-, -CHR^{8a}-, -CR^{8b}R^{8c}- oder eine -C(O)-Gruppe bedeuten, wobei
R^{8a}/R^{8b}/R^{8c}
jeweils unabhängig voneinander eine C₁₋₅-Alkylgruppe, die durch 1-3 Fluoratome substituiert sein kann, eine Hydroxy-C₁₋₅-alkyl-, C₁₋₅-Alkyloxy-C₁₋₅-alkyl-, Amino-C₁₋₅-alkyl-, C₁₋₅-Alkylamino-C₁₋₅-alkyl-, Di-(C₁₋₅-alkyl)-amino-C₁₋₅-alkyl-, C₄₋₇-Cycloalkylenimino-C₁₋₅-alkyl-, Carboxy-C₀₋₅-alkyl-, C₁₋₅-Alkyloxycarbonyl-C₀₋₅-alkyl-, Aminocarbonyl-C₀₋₅-alkyl-, C₁₋₅-Alkylaminocarbonyl-C₀₋₅-alkyl-, Di-(C₁₋₅-alkyl)-aminocarbonyl-C₀₋₅-alkyl- oder eine C₄₋₇-Cycloalkyleniminocarbonyl-C₀₋₅-alkyl-gruppe bedeutet,
oder zwei Reste R^{8b}/R^{8c} zusammen mit dem Ringkohlenstoffatom einen 3-, 4-, 5-, 6- oder 7-gliedrigen gesättigten Carbocyclus oder einen Cyclopenten-, Cyclohexen-, Oxetan-, Azetidin-, Thietan-, Tetrahydrofuran-, Pyrrolidin-, Tetrahydrothiophen-, Tetrahydropyran-, Piperidin-, Pentamethylensulfid-, Hexamethylenimin-, Hexahydropyridazin-, Tetrahydro-2(1H)-pyrimidinon-, [1,3]Oxazinan-2-on-ring bilden können,
wobei dessen Methylengruppen durch 1-2 C₁₋₃-Alkyl- oder CF₃-gruppen substituiert sein können, und/oder
dessen Methylengruppen, sofern sie nicht an ein Heteroatom gebunden sind, durch 1-2 Fluoratome substituiert sein können, und/oder
bei dem eine -CH₂-Gruppe neben einem Stickstoffatom durch eine -CO-Gruppe ersetzt sein kann, und/oder
dessen Iminogruppen jeweils durch eine C₁₋₃-Alkyl- oder C₁₋₃-Alkylcarbonyl-gruppe substituiert sein können, und/oder
bei dem das Schwefelatom zu einer Sulfoxid- oder Sulfongruppe oxidiert sein kann,
mit der Massgabe, dass ein durch R^{8b} oder R^{8c} eingebrachtes Heteroatom nicht durch nur ein Kohlenstoffatom von X in Formel (1) entfernt sein darf, und
insgesamt in Formel (IIa) oder (IIb) oder (IIc) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine CF₂-, Sulfen-, Sulfon- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂-, C₃₋₆-Cycloalkyl-, C₄₋₆-Cycloalkenyl-, Oxetan-3-yl-, Tetrahydrofuran-3-yl-, Benzyl-, C₁₋₅-Alkyl-carbonyl-, Trifluormethylcarbonyl-, C₃₋₆-Cycloalkyl-carbonyl-, C₁₋₅-Alkylsulfonyl-, C₃₋₆-Cycloalkyl-sulfonyl-, Aminocarbonyl-, C₁₋₅-Alkylaminocarbonyl-, Di-(C₁₋₅-alkyl)-aminocarbonyl-, C₁₋₅-Alkyloxycarbonyl-, C₄₋₇-Cycloalkyleniminocarbonyl-gruppe bedeutet,
wobei die in den voranstehend genannten Gruppen befindlichen Methylen- und Methylgruppen zusätzlich durch eine C₁₋₃Alkyl-, Carboxy-, C₁₋₅ -Alkoxycarbonylgruppe substituiert sein können, oder durch eine Hydroxy-, C₁₋₅-Alkyloxy-, Amino-, C₁₋₅-Alkylamino-, C₁₋₅-Dialkylamino- oder C₄₋₇-Cycloalkyleniminogruppe substituiert sein können, sofern die Methylen- oder Methylgruppen nicht direkt an ein Heteroatom aus der Gruppe O, N oder S gebunden sind, und/oder ein bis drei Wasserstoffatome durch Fluoratome ersetzt sein können, sofern die Methylen- oder Methylgruppen nicht direkt an ein Heteroatom aus der Gruppe O, N oder S gebunden sind,
und in dem
A¹ entweder N oder CR¹⁰ bedeutet,
A² entweder N oder CR¹¹ bedeutet,
A³ entweder N oder CR¹² bedeutet,
A⁴ entweder N oder CR¹² bedeutet,
A⁵ NH, Schwefel oder Sauerstoff bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, C₂₋₅ -Alkenyl-, C₂₋₅-Alkinyl-, eine Phenyl-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkylenimino-gruppe bedeuten, und
-L-E-G-J- eine -C-C-C-C-Gruppe bedeutet, die durch R⁴ und R⁵ substituiert sein kann, und
L¹ eine -C(O)-Gruppe bedeutet, und
R⁴ ein Wasserstoffatom oder
eine geradkettige oder verzweigte C₁-₆-Alkyl-, C₂₋₆-Alkenyl- oder C₂₋₆-Alkinylgruppe bedeutet,
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkyl-, C₂₋₆-Alkenyl- oder C₂₋₆-Alkinylgruppe gegebenenfalls jeweils unabhängig voneinander durch ein bis zwei Substituenten ausgewählt aus einer C₃₋₅-Cycloalkylgruppe, einer Nitril-, Hydroxy- oder C₁₋₅-Alkyloxygruppe, wobei die Wasserstoffatome der C₁₋₅-Alkyloxygruppe gegebenenfalls ganz oder teilweise durch Fluoratome ersetzt sein können, einer Allyloxy-, Propargyloxy-, Benzyloxy-, C₁₋₅-Alkylcarbonyloxy-, C₁₋₅-Alkyloxycarbonyloxy-, Carboxy-C₁₋₅-alkyloxy-, C₁₋₅-Alkyloxycarbonyl-C₁₋₅-alkyloxy-, Mercapto-, C₁₋₅-Alkylsulfanyl-, C₁₋₅-Alkylsulfinyl-, C₁₋₅-Alkylsulfonyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇Cycloalkyleniminocarbonyl-, Aminosulfonyl-, C₁₋₅-Alkylaminosulfonyl-, Di-(C₁₋₅-alkyl)-aminosulfonyl-, C₄₋₇-Cycloalkyleniminosulfonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe, oder einer Morpholinyl-, Thiomorpholinyl-, Pyrrolidinyl-, Piperidinyl-, Piperazinyl-, Tetrahydrofuranyl-, Tetrahydropyranyl-gruppe substituiert sein können, wobei die vorgenannten Carbo- und Hetero-cyclen im Ring jeweils durch 1-4 C₁₋₃-Alkyl- oder C₁₋₃-Alkylcarbonylgruppen oder jeweils durch 1-2 Oxogruppen substituiert sein können, und/oder
wobei die Wasserstoffatome der sp²-hybridisierten Kohlenstoffatome der geradkettigen oder verzweigten C₂₋₆-Alkenyl-gruppe gegebenenfalls ganz oder teilweise durch Fluoratome ersetzt sein können, oder
eine Nitril-, Carboxy-, Aminocarbonyl-, C₁₋₅-Alkylaminocarbonyl-, C₃₋₆-Cycloalkylaminocarbonyl-, Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, oder
eine Phenyl-, mono- oder bicyclische Heteroaryl-, Phenyl-C₁₋₅-alkyl- oder mono- oder bicyclische Heteroaryl-C₁₋₅-alkylgruppe,
die im Phenyl- oder Heteroarylteil gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein kann,
bedeutet,
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₃-alkyloxy-, Heteroaryl-C₀₋₃-alkyl-oxy-, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Morpholinyl-, Thiomorpholinyl-, Pyrrolidinyl-, Piperidinyl-, Piperazinyl-, Tetrahydrofuranyl-, Tetrahydropyranyl, Dimethylaminocarbonyl-, C₁₋₅-Alkyloxycarbonyl-, Carboxy-, Methyl-, Hydroxy-, Methoxy- oder Aminosubstituiert sein können, und
die vorgenannten Phenyl- oder Heteroarylreste gegebenenfalls ein- bis dreifach durch gleiche oder unterschiedliche Substituenten ausgewählt aus der Gruppe bestehend aus Fluor-, Chlor-, Brom-und Iodatomen, und C₁₋₅-Alkyl-, Trifluormethyl-, Amino-, C₁₋₅-alkyl-amino-, Di-(C₁₋₅-alkyl)-amino-, Hydroxy-, C₁₋₅-Alkyloxy-, Mono-, Di- oder Trifluormethoxy-, Carboxy- und C₁₋₅-Alkyloxycarbonylgruppe, substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O- Bindung bilden, ausgeschlossen ist, und
R⁵ ein Wasserstoffatom, eine C₁₋₅Alkyl-, C₂₋₅Alkenyl- oder C₂₋₅Alkinyl- oder eine Phenyl-C₀₋₅Alkylgruppe bedeutet, wobei die Alkylgruppe durch eine Hydroxy-, Methoxy-, Hydroxycarbonyl- oder C₁₋₅Alkoxycarbonyl-gruppe substituiert sein kann,
oder sofern R⁵ mit G verknüpft ist auch eine Hydroxy- oder Methoxygruppe darstellen kann, oder
R⁴ und R⁵ sofern sie an das selbe Kohlenstoffatom gebunden sind, zusammen mit dem Kohlenstoffatom eine -C=O Gruppe, oder eine -CF₂ Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus oder einen einfach ungesättigten 5-7 gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-,-N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂-O-Gruppe ersetzt sein können, und/oder
wobei 1 bis 3 Kohlenstoffatome dieser 3-7-gliedrigen Cyclen gegebenenfalls unabhängig voneinander durch jeweils ein oder zwei Fluoratome oder eine oder zwei C₁₋₅-Alkyl-gruppen oder eine Hydroxy-, C₁₋₅-Alkyloxy-, C₁₋₅-Alkylcarbonyloxy-, Amino-, C₁₋₅-Alkylamino-, Di-(C₁₋₅-alkyl)-amino-, C₄₋₇-Cycloalkylenimino-, C₁₋₅-Alkylcarbonylamino-, C₃₋₆-Cycloalkylcarbonylamino-, Nitril-, Carboxy-C₁₋₅-alkyl-, C₁₋₅-Alkyloxycarbonyl-C₁₋₅-alkyl-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl- oder C₄₋₇-Cycloalkyleniminocarbonylgruppe substituiert sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O- Bindung bilden,
ausgeschlossen ist, und
L² eine -C(O)-Gruppe bedeutet, und
M einen gegebenenfalls durch R² und R³ substituierten Phenyl-, Pyridyl-, Thienyl- oder Furyl-Ring bedeutet, in dem
R² ein Fluor-, Chlor-, Brom- oder Jod-atom oder eine Methyl-, Ethyl-, Vinyl-, Methoxy-, Ethinyl-, Cyano- oder -C(O)NH₂-Gruppe darstellt, und
R³ ein Wasserstoff-, Fluor-, Chlor-, Brom- oder Jod-atom oder eine Hydroxy-, Methoxy-, Trifluormethoxy-gruppe, oder eine gegebenenfalls durch Fluoratome substituierte C₁₋₃-Alkyl-gruppe, oder eine Cyano-, Amino- oder NH₂C(O)-Gruppe darstellt,
wobei, soweit nichts anderes erwähnt wurde, unter dem voranstehend in den Definitionen erwähnten Ausdruck "Heteroarylgruppe" eine monocyclische 5- oder 6-gliedrige Heteroarylgruppe zu verstehen ist, wobei
die 6-gliedrige Heteroarylgruppe ein, zwei oder drei Stickstoffatome, und
die 5-gliedrige Heteroarylgruppe eine gegebenenfalls durch eine C₁₋₃-Alkylgruppe substituierte Iminogruppe, ein Sauerstoff- oder Schwefelatom, oder
eine gegebenenfalls durch eine C₁₋₃-Alkylgruppe substituierte Iminogruppe oder ein Sauerstoff- oder Schwefelatom und zusätzlich ein oder zwei Stickstoffatome, oder
eine gegebenenfalls durch eine C₁₋₃-Alkylgruppe substituierte Iminogruppe und drei Stickstoffatome,
enthält,
und außerdem an die vorstehend erwähnten monocyclischen Heteroarylgruppen über zwei benachbarte Kohlenstoffatome ein gegebenenfalls durch ein Fluor-, Chlor- oder Bromatom, eine C₁₋₃-Alkyl-, Hydroxy-, C₁₋₃-Alkyloxygruppe, Amino-, C₁₋₃-Alkylamino-, Di-(C₁₋₃-alkyl)-amino- oder C₃₋₆-Cycloalkyleniminogruppe substituierter Phenylring ankondensiert sein kann,
und die Bindung über ein Stickstoffatom oder über ein Kohlenstoffatom des heterocyclischen Teils oder eines ankondensierten Phenylrings erfolgt,
und wobei, soweit nichts anderes erwähnt wurde, unter dem voranstehend in den Definitionen erwähnten Ausdruck "Halogenatom" ein Atom aus der Gruppe Fluor, Chlor, Brom und Iod zu verstehen ist,
und wobei die in den voranstehend erwähnten Definitionen enthaltenen Alkyl-, Alkenyl-, Alkinyl- und Alkyloxygruppen, die mehr als zwei Kohlenstoffatome aufweisen, soweit nichts anderes erwähnt wurde, geradkettig oder verzweigt sein können und die Alkylgruppen in den voranstehend genannten dialkylierten Resten, beispielsweise die Dialkylaminogruppen, gleich oder verschieden sein können,
und wobei die Wasserstoffatome der in den voranstehend erwähnten Definitionen enthaltenen Methyl- oder Ethylgruppen, sofern nichts anderes erwähnt, ganz oder teilweise durch Fluoratome ersetzt sein können,
deren Tautomere, deren Enantiomere, deren Diastereomere, deren Gemische und deren Salze.

2. Verbindungen der allgemeinen Formel (I) nach Anspruch 1, in denen D, E, G, J, L, L¹, L² und M wie in Anspruch 1 beschrieben definiert sind, und in der
R⁴ ein Wasserstoffatom oder
eine geradkettige oder verzweigte C₁₋₆-Alkylgruppe bedeutet,
wobei die Wasserstoffatome der Methylen- und/oder Methylfragmente der geradkettigen oder verzweigten C₁₋₆-Alkylgruppe gegebenenfalls ganz oder teilweise durch Fluoratome ersetzt sein können, und/oder
wobei die Wasserstoffatome der Methylen- und/oder Methylfragmente der geradkettigen oder verzweigten C₁₋₆-Alkylgruppe gegebenenfalls jeweils unabhängig voneinander durch einen Substituenten ausgewählt aus einer Hydroxy-, C₁₋₅-Alkyloxy-, Carboxy-, C₁₋₅-Alkyloxycarbonyl-, Aminocarbonyl-, C₁₋₅-Alkylaminocarbonyl-, Di-(C₁₋₅-alkyl)-aminocarbonyl-, C₄₋₇-Cycloalkyleniminocarbonyl-, Amino-, C₁₋₅-Alkylamino-, Di-(C₁₋₅-alkyl)-amino-, C₁₋₅-Alkylcarbonylamino-, C₁₋₅-Alkylsulfonylamino-, *N*-(C₁₋₅-Alkylsulfonyl)-C₁₋₅-alkylamino-, C₃₋₆-Cycloalkylcarbonylaminogruppe substituiert sein können, oder
eine Nitril-, Carboxy-, Aminocarbonyl-, C₁₋₅-Alkylaminocarbonyl-, C₃₋₆-Cycloalkylaminocarbonyl-,Di-(C₁₋₅-alkyl)-aminocarbonyl-, C₁₋₅-Alkyloxycarbonyl- oder eine C₄₋₇-Cycloalkyleniminocarbonylgruppe in der gegebenenfalls eine Methylengruppe durch ein Sauerstoff-, Schwefel- oder C₀₋₃-Alkyl-substituiertes Stickstoffatom ersetzt sein kann, bedeutet, und
oder sofern R⁴ mit G verknüpft ist auch ein Fluoratom oder eine Hydroxy-, C₁₋₅-Alkyl-oxy-, C₂₋₅-Alkenyl-oxy-, C₂₋₅-Alkinyl-oxy-, C₃₋₆-Cycloalkyl-oxy-, C₁₋₅-Alkylaminocarbonyloxy-, Di(C₁₋₅-alkyl)aminocarbonyloxy- oder C₄₋₇-Cycloalkyleniminocarbonyloxy-, Phenyl-C₀₋₂-alkyloxygruppe, Amino-, C₁₋₅-Alkylamino-, Di-(C₁₋₅-alkyl)-amino-, C₄₋₇-Cycloalkylenimino-, C₁₋₃-Acylamino-, (C₁₋₃-Acyl)C₁₋₃-alkylamino-, C₁₋₅-Alkyloxycarbonylamino-, C₁₋₅-Alkylaminocarbonylamino-, Di(C₁₋₅-alkyl)aminocarbonylamino- oder eine C₄₋₇-Cycloalkyleniminocarbonylamino-gruppe darstellen kann,
wobei die in den vorgenannten Alkyl- oder Cycloalkylresten vorhandenen Methyl oder Methylengruppen jeweils unabhängig voneinander durch einen Substituenten ausgewählt aus der Gruppe Dimethylaminocarbonyl-, C₁₋₅-Alkyloxycarbonyl-, Carboxy-, Methyl-, Hydroxy-, Methoxy- oder Amino- substituiert sein können,
mit der Maßgabe, dass zwei Heteroatome aus der Gruppe Sauerstoff und Stickstoff durch genau eine gegebenenfalls substituierte -CH₂-Gruppe voneinander getrennt sind, und/oder
dass zwei Atome eine -O-O- oder -S-O-Bindung bilden, ausgeschlossen ist, und
R⁵ ein Wasserstoffatom oder eine C₁₋₅Alkyl-, Allyl-, Propargyl- oder Benzylgruppe bedeutet, oder sofern R⁵ mit G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine -CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-7-gliedrigen Carbocyclus bilden können,
wobei eines der Kohlenstoffkettenglieder dieses Cyclus durch ein Sauerstoff- oder Schwefelatom oder eine -NH-, -N(C₁₋₅-Alkyl)-, N(C₁₋₄-Alkylcarbonyl)- oder eine Carbonyl-, Sulfinyl- oder Sulfonylgruppe ersetzt sein kann, und/oder
wobei zwei direkt benachbarte Kohlenstoffkettenglieder dieser C₄₋₇-Carbocyclen zusammen durch eine -C(O)NH-, -C(O)N(C₁₋₅-Alkyl)-, -S(O)₂NH-, oder -S(O)₂N(C₁₋₅-Alkyl)-Gruppe ersetzt sein können, und/oder
wobei vier direkt benachbarte Kohlenstoffkettenglieder dieser C₅₋₇-Carbocyclen zusammen durch eine -O-CH₂-CH₂O-Gruppe ersetzt sein können,
mit der Maßgabe, dass ein solcher, zusammen aus R⁴ und R⁵ gebildeter Cyclus,
in dem zwei Stickstoffatome oder ein Stickstoff und ein Sauerstoffatom im Cyclus durch genau eine gegebenenfalls substituierte -CH₂-Gruppe voneinander getrennt sind, und/oder
in der zwei Atome im Ring eine -O-O- oder -S-O-Bindung bilden,
ausgeschlossen ist,
deren Tautomere, deren Enantiomere, deren Diastereomere, deren Gemische und deren Salze.

3. Verbindungen der allgemeinen Formel (I) nach Anspruch 1 oder 2, in denen E, G, J, L, L¹, L² , M, R⁴ und R⁵ wie in Anspruch 1 oder 2 beschrieben definiert sind, und in der
D ein substituiertes bicyclisches Ringsystem der Formel (IIa) oder (IIb) 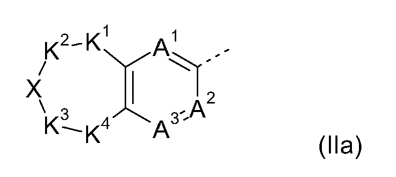 oder 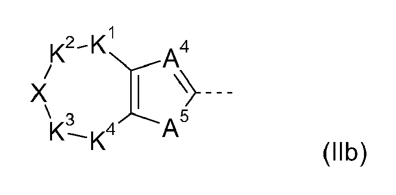 darstellt, in dem
K¹ und K⁴
jeweils unabhängig voneinander eine Bindung, eine -CH₂-, -CHR^{7a}-, -CR^{7b}R^{7c}- oder eine -C(O)-Gruppe bedeuten, und wobei
R^{7a}/R^{7b}/R^{7c}
jeweils unabhängig voneinander ein Fluoratom, eine Hydroxy-, C₁₋₅-Alkyloxy-, eine C₁₋₅-Alkylgruppe bedeutet,
wobei nicht gleichzeitig beide Reste R^{7b}/R^{7c} über ein Heteroatom an das Ringkohlenstoffatom gebunden sein können, außer - C(R^{7b}R^{7c})- entspricht einer -CF₂-Gruppe, oder
zwei Reste R^{7b}/R^{7c} zusammen mit dem Ringkohlenstoffatom einen 3-gliedrigen Carbocyclus bilden können,
mit der Maßgabe, dass K¹ und K⁴ gleichzeitig eine Bindung bedeuten, ausgeschlossen ist, und
K² und K³
jeweils unabhängig voneinander eine -CH₂-, -CHR^{8a}-, -CR^{8b}R^{8c}- oder eine -C(O)- Gruppe bedeuten, wobei
R^{8a}/R^{8b}/R^{8c}
jeweils unabhängig voneinander eine C₁₋₅-Alkylgruppe bedeutet, und/oder
zwei Reste R^{8b}/R^{8c} zusammen mit dem Ringkohlenstoffatom einen 3-gliedrigen gesättigten Carbocyclus bilden können
und
insgesamt in den Formeln (IIa) oder (IIb) maximal vier Reste ausgewählt aus R^{7a}, R^{7b}, R^{7c}, R^{8a}, R^{8b} und R^{8c} vorhanden sein dürfen, und
X ein Sauerstoff- oder Schwefelatom, eine -CF₂- oder eine NR¹-Gruppe bedeutet, in der
R¹ ein Wasserstoffatom oder eine Hydroxy-, C₁₋₃-Alkyloxy-, Amino-, C₁₋₃-Alkylamino-, Di-(C₁₋₃-alkyl)-amino-, eine C₁₋₅-Alkyl-, C₂₋₅-Alkenyl-CH₂-, C₂₋₅-Alkinyl-CH₂- oder eine C₃₋₆-Cycloalkylgruppe bedeutet,
und in dem
A¹ entweder N oder CR¹⁰ bedeutet,
A² entweder N oder CR¹¹ bedeutet,
A³ entweder N oder CR¹² bedeutet,
A⁴ entweder N oder CR¹² bedeutet,
A⁵ NH, Schwefel oder Sauerstoff bedeutet,
wobei R¹⁰, R¹¹ und R¹² jeweils unabhängig voneinander
ein Wasserstoff-, Fluor-, Chlor-, Brom- oder lodatom, oder eine C₁₋₅-Alkyl-, CF₃-, eine Cyano-, Carboxy-, C₁₋₅-Alkyloxycarbonyl-, Hydroxy-, C₁₋₃-Alkyloxy-, CF₃O-, CHF₂O-, CH₂FO-, Amino-, C₁₋₅-Alkylamino-, Di-(C₁₋₅-Alkyl)-amino- oder C₄₋₇-Cycloalkyleniminogruppe bedeuten,
deren Tautomere, deren Enantiomere, deren Diastereomere, deren Gemische und deren Salze.

4. Verbindungen der allgemeinen Formel (I) nach einem der Ansprüche 1 bis 3, in denen
X eine NR¹-Gruppe bedeutet, in der
R¹ ein Wasserstoffatom oder eine C₁₋₅-Alkyl-, Allyl- oder Cyclopropylgruppe bedeutet, und
A¹ CR¹⁰ bedeutet,
A² CR¹¹ bedeutet,
A³ CR¹² bedeutet,
A⁴ entweder N oder CR¹² bedeutet,
A⁵ Schwefel bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

5. Verbindungen der allgemeinen Formel (I) nach einem der Ansprüche 1 bis 4, in denen
D ein substituiertes bicyclisches Ringsystem der Formel 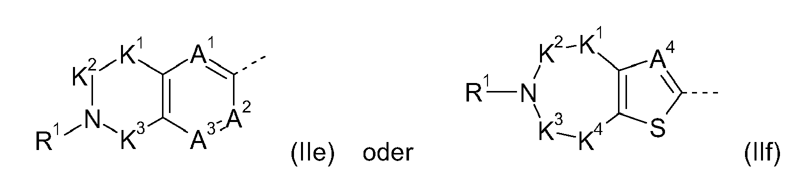 darstellt, in dem
K¹ eine -CH₂-, -CHR^{7a}-, oder eine -CR^{7b}R^{7c}- Gruppe bedeutet, und K² und K³
jeweils unabhängig voneinander eine -CH₂-, -CHR^{8a}-, oder eine - CR^{8b}R^{8c}- Gruppe bedeuten, wobei
R^{8a}/R^{8b}/R^{8c}
jeweils unabhängig voneinander eine C₁₋₅-Alkylgruppe bedeutet, und
K⁴ eine Bindung, eine -CH₂-, -CHR^{7a}-, oder eine -CR^{7b}R^{7c}- Gruppe bedeutet,
wobei
R^{7a} eine C₁₋₅-Alkylgruppe bedeutet und
R^{7b}/R^{7c} jeweils unabhängig voneinander eine Hydroxy-, C₁₋₅-Alkyloxy- oder eine C₁₋₅-Alkylgruppe bedeutet, wobei nicht gleichzeitig beide Reste R^{7b}/R^{7c} über ein Sauerstoffatom an das Ringkohlenstoffatom gebunden sein können, und
insgesamt in den Formeln (IIe) oder (IIf) maximal vier Reste ausgewählt aus R^{7a} R^{7b}, R^{7c}, R^{8a}, R^{8b} und R^{8c} vorhanden sein dürfen, und
R¹ ein Wasserstoffatom oder eine C₁₋₃-Alkyl-, Allyl- oder Cyclopropylgruppe bedeutet, und in denen
A¹ CR¹⁰ bedeutet,
A² CR¹¹ bedeutet,
A³ CR¹² bedeutet,
A⁴ entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten, und
-L-E-G-J- eine -C-C-C-C-Gruppe bedeutet, die durch R⁴ und R⁵ substituiert sein kann, und
R⁴ ein Wasserstoffatom oder
eine geradkettige oder verzweigte C₁₋₃-Alkylgruppe bedeutet, wobei die Wasserstoffatome der Methylen- und/oder Methylfragmente der geradkettigen oder verzweigten C₁₋₆-Alkylgruppe gegebenenfalls unabhängig voneinander durch einen Substituenten ausgewählt aus einer Hydroxy-, C₁₋₅-Alkyloxy-, Carboxy-, C₁₋₅-Alkyloxycarbonyl-gruppe substituiert sein können, oder
sofern R⁴ mit G verknüpft ist, auch ein Fluoratom oder eine Hydroxy-, Methoxy-, C₃₋₅-Alkenyl-oxy-, C₂₋₅-Alkyl-oxy-, C₃₋₆-Cycloalkyl-oxy-, Benzyloxy-, C₁₋₅-Alkylaminocarbonyloxy-, Di(C₁-₅-alkyl)aminocarbonyloxy- oder eine C₄₋₇-Cycloalkyleniminocarbonyloxygruppe darstellen kann,
mit der Maßgabe,
dass zwei Heteroatome aus der Gruppe Sauerstoff und Stickstoff durch genau eine gegebenenfalls substituierte -CH₂-Gruppe voneinander getrennt sind,
ausgeschlossen ist, und
R⁵ ein Wasserstoffatom oder eine C₁₋₅ Alkyl-, Allyl-, Benzyl- oder Phenylgruppe bedeutet, oder sofern R⁵ mit G verknüpft ist, auch eine Hydroxy- oder Methoxy-gruppe darstellen kann, oder
R⁴ und R⁵ sofern sie an das selbe Kohlenstoffatom gebunden sind, zusammen mit dem Kohlenstoffatom eine -C=O-Gruppe, oder eine -CF₂- Gruppe bilden können, oder
R⁴ und R⁵ sofern sie an das selbe Kohlenstoffatom oder an zwei benachbarte Kohlenstoffatome gebunden sind, zusammen mit dem oder den Kohlenstoffatomen einen 3-6-gliedrigen Carbocyclus bilden können,
wobei vier direkt benachbarte Kohlenstoffkettenglieder dieser C₅₋₆-Carbocyclen zusammen durch eine -O-CH₂-CH₂O-Gruppe ersetzt sein können,
deren Tautomere, deren Enantiomere, deren Diastereomere, deren Gemische und deren Salze.

6. Verbindungen der allgemeinen Formel (I) nach einem der Ansprüche 1 bis 5, in denen
D ein substituiertes bicyclisches Ringsystem der Formel 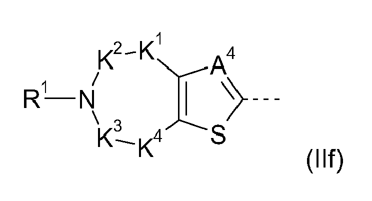 darstellt, in dem
K¹ eine -CH₂-, -CHR^{7a}-, oder eine -CR^{7b}R^{7c}- Gruppe bedeutet, und
K² und K³ jeweils unabhängig voneinander eine -CH₂-, -CHR^{8a}-, oder eine - CR^{8b}R^{8c}- Gruppe bedeuten, wobei
R^{8a}/R^{8b}/R^{8c}
jeweils unabhängig voneinander eine C₁₋₅-Alkylgruppe bedeutet, und
K⁴ eine Bindung, eine -CH₂-, -CHR^{7a}-, oder eine -CR^{7b}R^{7c}- Gruppe
bedeutet,
wobei
R^{7a} eine C₁₋₅-Alkylgruppe bedeutet, und
R^{7b}/R^{7c} jeweils unabhängig voneinander eine Hydroxy-, C₁₋₅-Alkyloxy- oder eine C₁₋₅-Alkylgruppe bedeutet,
wobei nicht gleichzeitig beide Reste R^{7b}/R^{7c} über ein Sauerstoffatom an das Ringkohlenstoffatom gebunden sein können,
und
insgesamt in der Formel (IIf) maximal vier Reste ausgewählt aus R^{7a}, R^{7b}, R^{7c}, R^{8a}, R^{8b}, und R^{8c} vorhanden sein dürfen, und
R¹ ein Wasserstoffatom oder eine C₁₋₃-Alkyl- oder Cyclopropylgruppe bedeutet, und in denen
A¹ CR¹⁰ bedeutet,
A² CR¹¹ bedeutet,
A³ CR¹² bedeutet,
A⁴ entweder N oder CR¹² bedeutet,
wobei R¹⁰, R¹¹ und R¹² jeweils unabhängig voneinander
ein Wasserstoff-, Fluor- oder Chloratom, oder eine Methyl-, CF₃-, Hydroxy-, Methoxy-, CF₃O-, CHF₂O-, CH₂FO-Gruppe bedeuten,
deren Tautomere, deren Enantiomere, deren Diastereomere, deren Gemische und deren Salze.

7. Verbindungen der allgemeinen Formel (I) nach einem der Ansprüche 1 bis 6, in denen
M einen Thiophen-2-yl-Ring der Formel 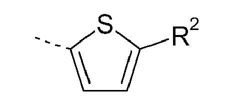 bedeutet, in dem
R² ein Chlor-, Bromatom oder eine Ethinyl-Gruppe darstellt,
deren Tautomere, deren Enantiomere, deren Diastereomere, deren Gemische und deren Salze.

8. Physiologisch verträgliche Salze der Verbindungen gemäß einem der Ansprüche 1 bis 7.

9. Arzneimittel, enthaltend eine Verbindung nach mindestens einem der Ansprüche 1 bis 7 oder ein physiologisch verträgliches Salz gemäß Anspruch 8, neben gegebenenfalls einem oder mehreren inerten Trägerstoffen und/oder Verdünnungsmitteln.

10. Verwendung einer Verbindung nach mindestens einem der Ansprüche 1 bis 7 oder eines physiologisch verträglichen Salzes gemäß Anspruch 8 zur Herstellung eines Arzneimittels mit einem inhibitorischen Effekt auf Faktor Xa und/oder einem inhibitorischen Effekt auf verwandte Serinproteasen.

11. Verfahren zur Herstellung eines Arzneimittels gemäß Anspruch 9, **dadurch gekennzeichnet, dass** auf nichtchemischem Wege eine Verbindung nach mindestens einem der Ansprüche 1 bis 7 oder ein physiologisch verträgliches Salz gemäß Anspruch 8 in einen oder mehrere inerte Trägerstoffe und/oder Verdünnungsmittel eingearbeitet wird.
